(19) Europäisches Patentamt
European Patent Office
Office européen des brevets

(11) Numéro de publication : **0 395 526 B1**

(12) # FASCICULE DE BREVET EUROPEEN

(45) Date de publication du fascicule du brevet : **02.03.94 Bulletin 94/09**

(51) Int. Cl.$^5$ : **C07D 277/68**

(21) Numéro de dépôt : **90401147.5**

(22) Date de dépôt : **27.04.90**

(54) **Nouveaux dérivés benzothiazolinoniques, leur procédé de préparation et les compositions pharmaceutiques qui les contiennent.**

(30) Priorité : **28.04.89 FR 8905655**

(43) Date de publication de la demande : **31.10.90 Bulletin 90/44**

(45) Mention de la délivrance du brevet : **02.03.94 Bulletin 94/09**

(84) Etats contractants désignés : **AT BE CH DE DK ES FR GB GR IT LI LU NL SE**

(56) Documents cités :
**FR-A- 2 244 506**
**FR-A- 2 244 507**
**FR-A- 2 409 991**
**CHEMICAL ABSTRACT, vol. 103, no. 11, 16 septembre 1985, page 609, résumé no. 87865c, Columbus, Ohio, US;**
**CHEMICAL ABSTRACTS, vol. 104, no. 3, 20 janvier 1986, page 483, résumé no. 19575d, Columbus, Ohio, US;**

(73) Titulaire : **ADIR ET COMPAGNIE**
**1 rue Carle Hébert**
**F-92415 Courbevoie Cédex (FR)**

(72) Inventeur : **Lesieur, Daniel**
**20 rue de Verdun**
**F-59147 Gondecourt (FR)**
Inventeur : **Lespagnol, Charles**
**115 avenue Pasteur**
**F-59130 Lambersart (FR)**
Inventeur : **Yous, Said**
**3 rue du Pr. Laguesse**
**F-59045 Lille Cédex (FR)**

**EP 0 395 526 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés de la benzothiazolinone, leurs préparations et les compositions pharmaceutiques qui les contiennent.

De nombreux dérivés de la benzothiazolinone ont été décrits en thérapeutique comme possédant des propriétés pharmacologiques très variées.

Le brevet JP 86143307 décrit notamment des alkyl-6 benzothiazolinones en tant que fongicides ; le brevet JP 85130574 décrit des amido-6 benzothiazolinones en tant que stimulants cardiaques ; enfin le brevet WO 8501289 décrit entre autres des acyl-6 benzothiazolinones en tant qu'anti-inflammatoires ou antithrombotiques.

Les brevets FR 7323280 et FR 8020861 décrivent quant à eux des acyl-6 benzoxazolinones utilisables en tant qu'analgésiques.

La demanderesse a maintenant découvert des dérivés benzothiazolinoniques doués d'une activité analgésique d'un niveau nettement plus intéressant que celui des dérivés décrits dans le brevet FR 7323280.

Les composés de la présente invention sont en effet complètement atoxiques et doués d'intenses propriétés analgésiques et possèdent par ailleurs une activité anti agrégante plaquettaire d'un bon niveau.

Enfin, de manière surprenante, ils présentent également des propriétés normolipémiantes très intéressantes en réduisant la cholestérolémie par abaissement des fractions athérogènes de faible densité (VLDL et LDL) et en améliorant la répartition du cholestérol plasmatique par augmentation du rapport HDL cholestérol/cholestérol total.

Plus spécifiquement, l'invention concerne les dérivés de formule générale (I):

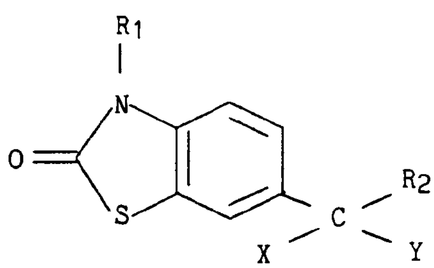

(I)

dans laquelle :

$R_1$ représente un atome d'hydrogène ou un groupement alkyle inférieur,

$R_2$ représente :

- . un groupement alkyle inférieur linéaire ou ramifié éventuellement substitué par un ou plusieurs atomes d'halogène ou par un ou plusieurs groupements alkoxy, hydroxy, phényle, acide carboxylique,
- . un groupement phényle éventuellement substitué par un ou plusieurs :
  - atomes d'halogène, ou
  - groupements alkyles inférieurs éventuellement substitués par un ou plusieurs atomes d'halogène, ou
  - groupements hydroxy ou alkoxy inférieurs,
- . un groupement alkényle inférieur linéaire ou ramifié éventuellement substitué par un groupement acide carboxylique, alkoxy, hydroxyle ou aryle,
- . un groupement thiényle-2, éventuellement substitué par un groupement alkyle inférieur linéaire ou ramifié,
- . un groupement furyle-2, éventuellement substitué par un groupement alkyle inférieur linéaire ou ramifié,
- . un groupement pyrrolyle-2, éventuellement substitué par un groupement alkyle inférieur linéaire ou ramifié,
- . un groupement pyridyle, éventuellement substitué par un groupement alkyle inférieur linéaire ou ramifié,

X représente un atome d'hydrogène,

Y représente un groupement hydroxyle,

ou bien X et Y représentent ensemble un atome d'oxygène,

le terme inférieur indiquant que les groupements ainsi qualifiés comptent de 1 à 6 atomes de carbone,

le terme inférieur qualifiant "alkényle" indique un groupement allant jusqu'à 6 atomes de carbone,

leurs énantiomères, diastéréoisomères et épimères ainsi que lorsque $R_1$ représente un atome d'hydrogène et $R_2$ comprend un groupement acide carboxylique, leurs sels d'addition à une base pharmaceutiquement acceptable ou lorsque R2 comprend un groupement aminé, leurs sels d'addition à un acide pharmaceutiquement

acceptable. Parmi les bases que l'on peut ajouter aux composés de formule (I) pour lesquels $R_1$ représente un atome d'hydrogène ou lorsque $R_2$ comprend un groupement acide carboxylique, on peut citer, à titre d'exemple, les hydroxydes de sodium, potassium, calcium ou des bases organiques comme la diéthylamine, la diéthanolamine, la triéthylamine, la benzylamine, la dicyclohexylamine, l'arginine, ou des carbonates de métaux alcalins ou alcalino terreux.

Parmi les acides que l'on peut ajouter aux composés de formule (I) pour lesquels R2 comprend un groupement aminé, on peut citer à titre d'exemple les acides chlohydrique, sulfurique, phosphorique, tartrique, malique, maléique, fumarique, oxalique, méthane-sulfonique, camphorique, citrique etc...

L'invention concerne, par exemple :

- les composés selon la formule (I), dans lesquels X et Y représentent ensemble un atome d'oxygène, leurs énantiomères, diastéréoisomères, et épimères ainsi que, lorsque $R_1$ représente un atome d'hydrogène et/ou $R_2$ comprend un groupement acide carboxylique, leurs sels d'addition à une base pharmaceutiquement acceptable, ainsi que lorsque $R_2$ comprend un groupement aminé, leurs sels d'addition à un acide pharmaceutiquement acceptable,
- les composés selon la formule (I), dans lesquels $R_1$ représente un atome d'hydrogène, leurs énantiomères, diastéréoisomères, et épimères ainsi que leurs sels d'addition à une base pharmaceutiquement acceptable, et lorsque $R_2$ comprend un groupement aminé, leurs sels d'addition à un acide pharmaceutiquement acceptable,
- les composés selon la formule (I), dans lesquels $R_1$ représente un groupement méthyle, leurs énantiomères, diastéréoisomères, et épimères ainsi que lorsque $R_2$ comprend un groupement acide carboxylique, leurs sels d'addition à une base pharmaceutiquement acceptable, et lorsque $R_2$ comprend un groupement aminé, leurs sels d'addition à un acide pharmaceutiquement acceptable,
- les composés selon la formule (I), dans lesquels $R_2$ représente un groupement phényle, leurs énantiomères, diastéréoisomères, épimères, ainsi que, lorsque $R_1$ représente un atome d'hydrogène, leurs sels d'addition à une base pharmaceutiquement acceptable,
- les composés selon la formule (I), dans lesquels $R_2$ représente un groupement alkyle, leurs énantiomères, diastéréoisomères, et épimères, atome d'hydrogène, ainsi que, lorsque $R_1$ représente un atome d'hydrogène, leurs sels d'addition à une base pharmaceutiquement acceptable,
- les composés selon la formule (I), qui est la benzoyl-6 benzothiazolinone, ainsi que ses sels d'addition à une base pharmaceutiquement acceptable,
- les composés selon la formule (I), dans lesquels $R_2$ représente un groupement phényle, ainsi que lorsque $R_1$ représente un atome d'hydrogène, leurs sels d'addition à une base pharmaceutiquement acceptable,
- les composés selon la formule (I), dans lesquels $R_2$ représente un groupement phényle substitué par un ou plusieurs atomes d'halogène, ainsi que, lorsque $R_1$ représente un atome d'hydrogène, leurs sels d'addition à une base pharmaceutiquement acceptable,
- les composés selon la formule (I), dans lesquels $R_2$ représente un groupement thiényle, ainsi que lorsque $R_1$ représente un atome d'hydrogène, leurs sels d'addition à une base pharmaceutiquement acceptable,
- les composés selon la formule (I), dans lesquels $R_2$ représente un groupement alkyle inférieur ou phényle substitué par un groupement hydroxyle, leurs énantiomères, diastéréoisomères, épimères ainsi que lorsque $R_1$ représente un atome d'hydrogène, leurs sels d'addition à une base pharmaceutiquement acceptable,
- les composés selon la formule (I), dans lesquels $R_2$ représente un groupement alkyle inférieur substitué par un atome d'halogène, leurs énantiomères, diastéréoisomères, épimères ainsi que, lorsque $R_1$ représente un atome d'hydrogène, leurs sels d'addition à une base pharmaceutiquement acceptable,
- les composés selon la formule (I), dans lesquels $R_2$ représente un groupement alkyle inférieur substitué par un groupement acide carboxylique, leurs énantiomères, diastéréoisomères et épimères ainsi que leurs sels d'addition à une base pharmaceutiquement acceptable,
- les composés selon la formule (I), dans lesquels $R_2$ représente un groupement alcényle inférieur substitué par un groupement acide carboxylique, leurs énantiomères, diastéréoisomères, et épimères ainsi que leurs sels d'addition à une base pharmaceutiquement acceptable,
- les composés selon la formule (I), dans lesquels $R_2$ représente un groupement pyridyle leurs énantiomères, diastéréoisomères et épimères ainsi que leurs sels d'addition à un acide pharmaceutiquement acceptable, et lorsque $R_1$ représente un atome d'hydrogène, leurs sels d'addition à une base pharmaceutiquement acceptable,
- les composés selon la formule (I), dans lesquels X représente un atome d'hydrogène, Y représente un groupement hydroxyle, leurs énantiomères, diastéréoisomères et épimères ainsi que, lorsque $R_1$ représente un atome d'hydrogène, leurs sels d'addition à une base pharmaceutiquement acceptable et lors-

que $R_2$ comprend un groupement aminé leurs sels d'addition à un acide pharmaceutiquement acceptable,

- les composés selon la formule (I),1 dans lesquels $R_2$ représente un groupement phényle alkyle inférieur leurs énantiomères, diastéréoisomères et épimères ainsi que lorsque $R_1$ représente un atome d'hydrogène leurs sels d'addition à une base pharmaceutiquement acceptable,
- et les composés selon la formule (I), dans lesquels $R_2$ représente un groupement phényle substitué par un ou plusieurs groupements choisis parmi alkyle inférieur, alkoxy inférieur, hydroxy, trifluorométhyle leurs isomères, diastéréoisomères, épimères, ainsi que lorsque $R_1$ représente un atome d'hydrogène, leurs sels d'addition à une base pharmaceutiquement acceptable.

L'invention s'étend aussi au procédé d'obtention des composés de formule générale (I), caractérisé en ce que l'on utilise comme matière première un dérivé de formule (II) :

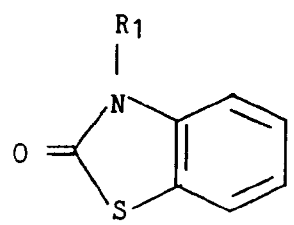

(II)

dans laquelle $R_1$ a la même signification que dans la formule (I),
obtenu, par exemple, par réaction de l'orthoaminothiophénol sur l'urée suivie, lorsque $R_1$ est différent de H, d'une alkylation à l'azote,
que l'on soumet, en fonction de la nature de $R_2$ :

* ou bien à l'action d'un chlorure d'acide de formule (III) :

$R_2$ - CO - Cl (III)

dans laquelle $R_2$ a la même signification que dans la formule (I), ou bien de l'anhydride d'acide ou de la lactone correspondant,
dans les conditions classiques de la réaction de Friedel-Crafts en utilisant préférentiellement le chlorure d'aluminium en présence de diméthylformamide selon les conditions de THYES et Coll. (J. Med. Chem., 1983, 26, 6, 800-807),
pour obtenir un dérivé de formule (I/a) :

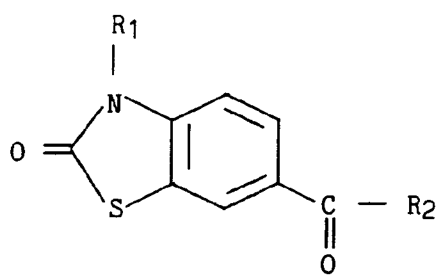

(I/a)

cas particulier des dérivés de formule (I), dans laquelle :

$R_1$ et $R_2$ ont la même signification que dans la formule (I),

X et Y représentent ensemble un atome d'oxygène,

* ou bien à l'action d'un acide de formule (V) :

**$R_2$ - $CO_2H$ (V)**

dans laquelle $R_2$ a la même signification que dans la formule (I),
ou du chlorure d'acide ou de l'anhydride d'acide ou de la lactone correspondant en présence d'acide polyphosphorique selon les conditions décrites dans le brevet Fr. 73.23280,
pour obtenir un dérivé de formule (I/a) :

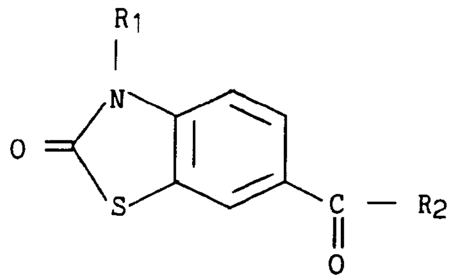

(I/a)

cas particulier des dérivés de formule (I), dans laquelle :

$R_1$ et $R_2$ ont la même signification que dans la formule (I),

X et Y représentent ensemble un atome d'oxygène,

dérivé de formule (I/a) que l'on purifie éventuellement par une technique classique de purification, et que, l'on peut, dans les cas où $R_1$ représente un atome d'hydrogène, ou dans les cas où $R_2$ comprend un groupement acide carboxylique ou un groupement amine, si on le désire, salifier respectivement par une base ou un acide pharmaceutiquement acceptable,

et qui, lorsque $R_2$ représente un groupement aryle substitué par un ou plusieurs groupements alkoxy inférieurs peut être soumis, si on le désire, à l'action d'un acide fort, pour conduire à un dérivé de forumule de (I/z) :

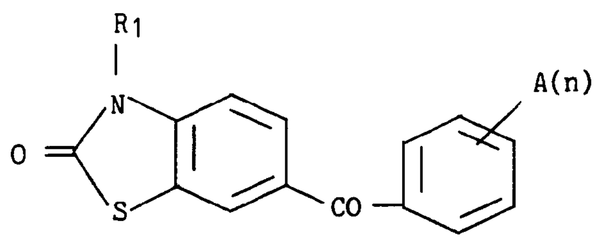

(I/z)

dans laquelle $R_1$ a la même signification que dans la formule (I), A représente un groupement hydroxy et n est un nombre entier compris entre 1 et 5, la position du ou des groupement(s) hydroxy étant identique à celle du (des) groupement(s) alkoxy inférieur du produit de formule (I) soumis à l'action de l'acide, produit de formule (I/z) que l'on purifie éventuellement par une technique classique et que, l'on peut, dans les cas où $R_1$ représente un atome d'hydrogène, si on le désire, salifier par une base pharmaceutiquement acceptable,

dérivé de formule (I/a) ou (I/z) qui, si on le souhaite, peut être soumis à l'action d'un agent d'hydrogénation préférentiellement choisi parmi un hydrure mixte de métal alcalin comme par exemple le borohydrure de sodium, préférentiellement en présence d'alcool aliphatique inférieur ou en milieu acide, pour conduire après éventuelle neutralisation du milieu réactionnel à un dérivé de formule (I/b) :

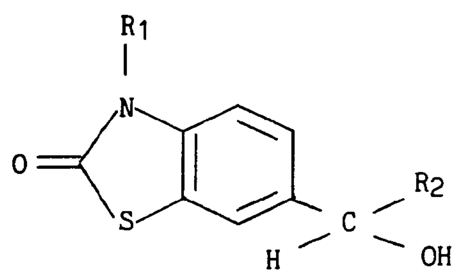

(I/b)

cas particulier des dérivés de formule (I) dans laquelle :

$R_1$ et $R_2$ ont la même signification que dans la formule (I),

X représente un atome d'hydrogène,

Y représente un groupement hydroxyle,

dont on sépare, si on le souhaite, les isomères, par une technique classique de séparation et que l'on purifie éventuellement par une technique classique de purification, et que, dans les cas où $R_1$ représente un atome d'hydrogène, où dans les cas où $R_2$ comprend un groupement acide carboxylique ou un groupement amine, l'on peut, si on le désire, salifier respectivement par une base ou un acide pharmaceutiquement acceptable.

L'invention s'étend également au procédé de préparation de composés de formule (I/z) :

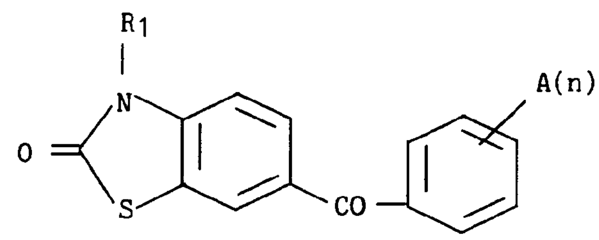

(I/z)

cas particulier des dérivés de formule (I) dans lequel R1 a la même signification que dans la formule (I), A représente un groupement hydroxy et n est un nombre entier compris entre 1 et 5 caractérisé en ce que l'on soumet un dérivé de formule (I) selon la revendication 1 dans lequel le groupement R2 signifie un radical phényle substitué par 1 à 5 groupements alkoxy inférieur à l'action d'un acide fort,
pour conduire à un dérivé de formule (I/z), la position des groupements hydroxy du dérivé ainsi obtenu étant identique à celle des groupements alkoxy inférieur du produit de formule (I) soumis à l'acide fort,
produit de formule (I/z) que l'on purifie éventuellement par une technique classique et que, l'on peut, dans le cas où $R_1$ représente un atome d'hydrogène, si on le désire, salifier par une base pharmaceutiquement acceptable.

L'invention s'étend également :

- au procédé de préparation de composés de formule (I) dans lesquels X et Y représentent ensemble un atome d'oxygène, leurs énantiomères, diastéréoisomères, et épimères ainsi que, lorsque $R_1$ représente un atome d'hydrogène et/ou $R_2$ comprend un groupement acide carboxylique, leurs sels d'addition à une base pharmaceutiquement acceptable, ainsi que lorsque $R_2$ comprend un groupement aminé, leurs sels d'addition à un acide pharmaceutiquement acceptable,
- au procédé de préparation de composés de formule (I) dans lesquels $R_1$ représente un atome d'hydrogène, leurs énantiomères, diastéréoisomères, et épimères ainsi que leurs sels d'addition à une base pharmaceutiquement acceptable, et lorsque $R_2$ comprend un groupement aminé, leurs sels d'addition à un acide pharmaceutiquement acceptable,
- au procédé de préparation de composés de formule (I), dans lesquels $R_1$ représente un groupement méthyle, leurs énantiomères, diastéréoisomères, et épimères ainsi que, lorsque $R_2$ comprend un groupement acide carboxylique, leurs sels d'addition à une base pharmaceutiquement acceptable, et lorsque $R_2$ comprend un groupement aminé, leurs sels d'addition à un acide pharmaceutiquement acceptable,
- au procédé de préparation de composés de formule (I) dans lesquels $R_2$ représente un groupement phényle, leurs énantiomères, diastéréoisomères, épimères, ainsi que, lorsque $R_1$ représente un atome d'hydrogène, leurs sels d'addition à une base pharmaceutiquement acceptable,
- au procédé de préparation de composés de formule (I) dans lesquels $R_2$ représente un groupement alkyle, leurs énantiomères, diastéréoisomères, et épimères, atome d'hydrogène, ainsi que, lorsque R1 représente un atome d'hydrogène, leurs sels d'addition à une base pharmaceutiquement acceptable,
- au procédé de préparation du composé de formule (I) qui est la benzoyl-6 benzothiazolinone, ainsi que ses sels d'addition à une base pharmaceutiquement acceptable,
- au procédé de préparation de composés de formule (I) dans lesquels $R_2$ représente un groupement phényle, ainsi que lorsque $R_1$ représente un atome d'hydrogène, leurs sels d'addition à une base pharmaceutiquement acceptable,
- au procédé de préparation de composés de formule (I) dans lesquels $R_2$ représente un groupement phényle substitué par un ou plusieurs atomes d'halogène, ainsi que, lorsque $R_1$ représente un atome d'hydrogène, leurs sels d'addition à une base pharmaceutiquement acceptable,
- au procédé de préparation de composés de formule (I) dans lesquels $R_2$ représente un groupement thiényle, ainsi que lorsque $R_1$ représente un atome d'hydrogène, leurs sels d'addition à une base pharmaceutiquement acceptable,
- au procédé de préparation de composés de formule (I) dans lesquels $R_2$ représente un groupement alkyle inférieur ou phényle substitué par un groupement hydroxyle, leurs énantiomères, diastéréoisomères, épimères ainsi que lorsque $R_1$ représente un atome d'hydrogène, leurs sels d'addition à une base pharmaceutiquement acceptable,
- au procédé de préparation de composés de formule (I) dans lesquels $R_2$ représente un groupement alky-

le inférieur substitué par un atome d'halogène, leurs énantiomères, diastéréoisomères, épimères ainsi que, lorsque $R_1$ représente un atome d'hydrogène, leurs sels d'addition à une base pharmaceutiquement acceptable,

- au procédé de préparation de composés de formule (I) dans lesquels $R_2$ représente un groupement alkyle inférieur substitué par un groupement acide carboxylique, leurs énantiomères, diastéréoisomères et épimères ainsi que leurs sels d'addition à une base pharmaceutiquement acceptable,
- au procédé de préparation de composés de formule (I) dans lesquels $R_2$ représente un groupement alcényle inférieur substitué par un groupement acide carboxylique, leurs énantiomères, diastéréoisomères, et épimères ainsi que leurs sels d'addition à une base pharmaceutiquement acceptable,
- au procédé de préparation de composés de formule (I) dans lesquels $R_2$ représente un groupement pyridyle leurs énantiomères, diastéréoisomères et épimères ainsi que leurs sels d'addition à un acide pharmaceutiquement acceptable, et lorsque $R_1$ représente un atome d'hydrogène, leurs sels d'addition à une base pharmaceutiquement acceptable,
- au procédé de préparation de composés de formule (I) dans lesquels X représente un atome d'hydrogène, Y représente un groupement hydroxyle, leurs énantiomères, diastéréoisomères et épimères ainsi que, lorsque $R_1$ représente un atome d'hydrogène, leurs sels d'addition à une base pharmaceutiquement acceptable et lorsque $R_2$ comprend un groupement aminé, leurs sels d'addition à un acide pharmaceutiquement acceptable,
- au procédé de préparation de composés de formule (I) dans lesquels $R_2$ représente un groupement phényle alkyle inférieur leurs énantiomères, diastéréoisomères et épimères ainsi que lorsque $R_1$ représente un atome d'hydrogène leurs sels d'addition à une base pharmaceutiquement acceptable,
- au procédé de préparation de composés de formule (I) dans lesquels $R_2$ représente un groupement phényle substitué par un ou plusieurs groupements choisis parmi alkyle inférieur, alkoxy inférieur, hydroxy, trifluorométhyle leurs isomères, diastéréoisomères, épimères, ainsi que lorsque $R_1$ représente un atome d'hydrogène, leurs sels d'addition à une base pharmaceutiquement acceptable,
- et au procédé de préparation de compositions pharmaceutiques contenant comme principe actif au moins un composé obtenu selon les procédés précédents en combinaison avec un ou plusieurs excipients ou véhicules inertes, non toxiques, pharmaceutiquement acceptables.

Les composés de formule (I) possèdent des propriétés pharmacologiques intéressantes.

L'étude pharmacologique des dérivés de l'invention a en effet montré qu'ils étaient peu toxiques, doués d'une activité analgésique et de propriétés normolipémiantes. Ce spectre d'activité rend donc les composés de la présente invention intéressants dans un certain nombre d'indications telles que algies rhumatismales, névralgies, lombosciatiques, névralgies cervico-brachiales, algies traumatiques telles que entorses, fractures, luxations, douleurs post-traumatiques, douleurs post-opératoires, douleurs dentaires, douleurs neurologiques telles que névralgies faciales, douleurs viscérales telles que coliques néphrétiques, dysménorrhées, chirurgie proctologique, douleurs de la sphère O.R.L., pancréatites, algies diverses, céphalées, douleurs des cancéreux, dans le cas d'hypercholestérolémies et hypertriglycéridémies endogènes isolées ou associées, ainsi que dans la prévention des accidents ischémiques artériels périphériques et cérébrovasculaires.

La présente invention a également pour objet les compositions pharmaceutiques contenant les produits de formule (I), seuls ou en combinaison avec un ou plusieurs excipients ou véhicules inertes non toxiques, pharmaceutiquement acceptables.

La présente invention a également pour objet une composition pharmaceutique contenant au moins un principe actif selon la formule (I) utilisable comme analgésique ou comme normolipémiant et dans le traitement des maladies artérielles ischémiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les sachets, les paquets, les gélules, les glossettes, les tablettes, les suppositoires, les crèmes, pommades, gels dermiques, etc...

La posologie utile varie selon l'âge et le poids du patient, la voie d'administration, la nature de l'indication thérapeutique ou des traitements éventuellement associés et s'échelonne entre 1 centigramme et 4 grammes par 24 heures.

Les exemples suivants illustrent l'invention.

### EXEMPLE 1 : BENZOYL-6 BENZOTHIAZOLINONE

A une solution de 0,04 mole de benzothiazolinone dans 150 g d'acide polyphosphorique, on ajoute lentement et sous agitation 0,05 mole d'acide benzoïque. Le milieu réactionnel est chauffé à 130 °C pendant 4 heures. Après refroidissement, le mélange est hydrolysé dans 10 volumes d'eau glacée.

Le précipité obtenu est essoré, lavé à l'eau jusqu'à neutralité du filtrat et séché.
Recristalliser dans de l'éthanol.

Rendement :80 %
Point de fusion : 216-217 °C
Infrarouge :
ν CO (thiocarbamate) : 1680 $cm^{-1}$
ν CO (cétone) : 1630 $cm^{-1}$

Microanalyse élémentaire :

| | C % | H % | N % | S % |
|---|---|---|---|---|
| Calculé | 65,86 | 3,55 | 5,49 | 12,56 |
| Trouvé | 65,69 | 3,60 | 5,39 | 12,63 |

**EXEMPLE 2 : METHYL-3 BENZOYL-6 BENZOTHIAZOLINONE**

Procéder de la même façon que dans l'exemple 1 en remplaçant la benzothiazolinone par la méthyl-3 benzothiazolinone et en maintenant l'agitation pendant 5 heures à 120 °C.
Recristalliser dans de l'éthanol absolu.

Rendement : 82 %
Point de fusion : 148 °C
Infrarouge :
ν CO (thiocarbamate) : 1685 $cm^{-1}$
ν CO (cétone) : 1635 $cm^{-1}$

Microanalyse élémentaire :

| | C % | H % | N % |
|---|---|---|---|
| Calculé | 66,89 | 4,12 | 5,20 |
| Trouvé | 66,69 | 4,14 | 5,22 |

**EXEMPLE 3 : (CHLORO-4 BENZOYL) -6 BENZOTHIAZOLINONE**

Procéder de la même façon que dans l'exemple 1 en remplaçant l'acide benzoïque par l'acide chloro-4 benzoïque et en maintenant l'agitation 1 heure 30 minutes à 145 °C.
Recristalliser dans de l'éthanol absolu.

Rendement : 75 %
Point de fusion : > 270 °C
Infrarouge :
ν CO (thiocarbamate) : 1730 $cm^{-1}$
ν CO (cétone) : 1630 $cm^{-1}$

Microanalyse élémentaire :

| | C % | H % | N % | Cl % |
|---|---|---|---|---|
| Calculé | 58,03 | 2,78 | 4,83 | 12,37 |
| Trouvé | 57,84 | 2,81 | 4,99 | 12,22 |

**EXEMPLE 4 : METHYL-3 (CHLORO-4 BENZOYL)- 6 BENZOTHIAZOLINONE**

Procéder de la même façon que dans l'exemple 3 en remplaçant la benzothiazolinone par la méthyl-3 benzothiazolinone et en maintenant l'agitation pendant 2 heures à 140 °C.

Recristalliser dans de l'éthanol.

Rendement : 78 %

Point de fusion : 169-170 °C

Infrarouge :

v CO (thiocarbamate) : 1700 $cm^{-1}$

v CO (cétone) : 1635 $cm^{-1}$

Microanalyse élémentaire :

| | C % | H % | N % | Cl % | S % |
|---|---|---|---|---|---|
| Calculé | 59,31 | 3,32 | 4,61 | 11,67 | 10,55 |
| Trouvé | 59,15 | 3,15 | 4,57 | 11,55 | 10,66 |

**EXEMPLE 5 : PROPIONYl-6 BENZOTHIAZOLINONE**

Procéder de la même façon que dans l'exemple 1 en remplaçant l'acide benzoïque par l'acide propionique et en maintenant l'agitation 4 heures à 100 °C.

Recristalliser dans du propanol.

Rendement : 30%

Point de fusion : 204-205 °C

Infrarouge :

v CO (thiocarbamate) : 1690 $cm^{-1}$

v CO (cétone) : 1650 $cm^{-1}$

Microanalyse élémentaire :

| | C % | H % | N % | S % |
|---|---|---|---|---|
| Calculé | 57,95 | 4,39 | 6,76 | 15,47 |
| Trouvé | 57,88 | 4,26 | 6,65 | 15,03 |

**EXEMPLE 6 : METHYL-3 PROPIONYL-6 BENZOTHIAZOLINONE**

Procéder de la même façon que dans l'exemple 5 en remplaçant la benzothiazolinone par la méthyl-3 benzothiazolinone et en maintenant l'agitation 2 heures 30 minutes à 90 °C.

Recristalliser dans de l'éthanol.

Rendement : 60 %

Point de fusion : 178 °C

Infrarouge : v CO : 1655 $cm^{-1}$ (thiocarbamate et cétone)

Microanalyse élémentaire :

| | C % | H % | N % |
|---|---|---|---|
| Calculé | 59,71 | 5,01 | 6,33 |
| Trouvé | 59,44 | 5,06 | 6,31 |

**EXEMPLE 7 : BUTYRYL-6 BENZOTHIAZOLINONE**

Procéder de la même façon que dans l'exemple 1 en remplaçant l'acide benzoïque par l'acide butyrique et en maintenant la température à 90°C pendant 4 heures.

Recristalliser dans de l'éthanol.

Rendement : 50 %

Point de fusion : 143-145°C

Infrarouge :

v CO (thiocarbamate) : 1685 $cm^{-1}$

v CO (cétone) : 1660 $cm^{-1}$

Microanalyse élémentaire :

| | C % | H % | N % | S % |
|---|---|---|---|---|
| Calculé | 59,70 | 4,97 | 6,33 | 14,49 |
| Trouvé | 59,31 | 5,05 | 6,30 | 14,52 |

**EXEMPLE 8 : METHYL-3 BUTYRYL-6 BENZOTHIAZOLINONE**

Procéder de la même façon que dans l'exemple 7 en remplaçant la benzothiazolinone par la méthyl-3 benzothiazolinone et en maintenant l'agitation pendant 3 heures à 100 °C.
Recristalliser dans de l'éthanol.

Rendement : 55 %
Point de fusion : 115-116 °C
Infrarouge :
v CO (thiocarbamate) : 1675 $cm^{-1}$
v CO (cétone) : 1660 $cm^{-1}$

Microanalyse élémentaire :

| | C % | H % | S % |
|---|---|---|---|
| Calculé | 61,25 | 5,57 | 13,63 |
| Trouvé | 61,43 | 5,64 | 13,82 |

**EXEMPLE 9 : VALERYL-6 BENZOTHIAZOLINONE**

Procéder de la même façon que dans l'exemple 1 en remplaçant l'acide benzoïque par l'acide valérique et en maintenant l'agitation pendant 2 heures 30 minutes à 100 °C.
Recristalliser dans de l'éthanol absolu.

Rendement :57 %
Point de fusion : 142-143 °C
Infrarouge :
v CO (thiocarbamate) : 1690 $cm^{-1}$
v CO (cétone) : 1665 $cm^{-1}$

Microanalyse élémentaire :

| | C % | H % | N % | S % |
|---|---|---|---|---|
| Calculé | 61,25 | 5,57 | 5,95 | 13,63 |
| Trouvé | 61,21 | 5,58 | 5,95 | 13,74 |

**EXEMPLE 10 : METHYL-3 VALERYL-6 BENZOTHIAZOLINONE**

Procéder de la même façon que dans l'exemple 9 en remplaçant la benzothiazolinone par la méthyl-3 benzothiazolinone et en maintenant l'agitation pendant 3 heures à 100 °C.
Recristalliser dans de l'éthanol absolu.

Rendement : 60 %
Point de fusion : 93-94 °C.
Infrarouge :
v CO (thiocarbamate) : 1680 $cm^{-1}$
v CO (cétone) : 1660 $cm^{-1}$

Microanalyse élémentaire :

| | C % | H % | S % |
|---|---|---|---|
| Calculé | 62,62 | 6,04 | 12,86 |
| Trouvé | 62,80 | 6,00 | 13,13 |

**EXEMPLE 11 : (THENOYL-2)-6 BENZOTHIAZOLINONE**

Procéder de la même façon que dans l'exemple 1 en remplaçant l'acide benzoïque par l'acide thiophène-2 carboxylique et en maintenant l'agitation 5 heures à 75 °C.
Recristalliser dans de l'éthanol absolu.

Rendement : 20 %
Point de fusion : 224° C
Infrarouge :
v CO (thiocarbamate) : 1735 $cm^{-1}$
v CO (cétone) : 1620 $cm^{-1}$

Microanalyse élémentaire :

| | C % | H % | N % |
|---|---|---|---|
| Calculé | 55,15 | 2,70 | 5,36 |
| Trouvé | 54,63 | 2,68 | 5,13 |

**EXEMPLE 12 : METHYL-3 (THENOYL-2)-6 BENZOTHIAZOLINONE**

Procéder de la même façon que dans l'exemple 11 en remplaçant la benzothiazolinone par la méthyl-3 benzothiazolinone et en maintenant l'agitation pendant 5 heures à 80 °C.
Recristalliser dans de l'éthanol absolu.

Rendement : 36 %
Point de fusion : 164-165°C
Infrarouge :
v CO (thiocarbamate) : 1660 $cm^{-1}$
v CO (cétone) : 1620 $cm^{-1}$

Microanalyse élémentaire :

| | C % | H % | S % |
|---|---|---|---|
| Calculé | 56,70 | 3,29 | 23,29 |
| Trouvé | 56,56 | 3,38 | 23,00 |

**EXEMPLE 13 : (HYDROXY-4 BUTYRYL)-6 BENZOTHIAZOLINONE**

Procéder de la même façon que dans l'exemple 1 en remplaçant l'acide benzoïque par la $\gamma$-butyrolactone et en maintenant l'agitation pendant 2 heures à 165 °C.
Recristalliser dans de l'acétate d'éthyle.

Rendement : 30 %
Point de fusion : 159-161 °C
Infrarouge :
v CO (thiocarbamate) : 1680 $cm^{-1}$
v CO (cétone) : 1660 $cm^{-1}$

Microanalyse élémentaire :

| | C % | H % | S % |
|---|---|---|---|
| Calculé | 55,67 | 4,67 | 13,51 |
| Trouvé | 55,68 | 4,68 | 13,44 |

**EXEMPLE 14 : METHYL-3 (HYDROXY-4 BUTYRYL)-6 BENZOTHIAZOLINONE**

Procéder de la même façon que dans l'exemple 13 en remplaçant la benzothiazolinone par la méthyl-3 benzothiazolinone.

**EXEMPLE 15 : ACETYL-6 BENZOTHIAZOLINONE**

A une solution contenant 0,5 mole de chlorure d'aluminium anhydre dans 0,20 mole de diméthylformamide, on ajoute 0,05 mole de benzothiazolinone puis lentement et sous agitation 0,06 mole de chlorure d'acide acétique.
Le milieu réactionnel est chauffé pendant 3 heures à 80 °C.
Après refroidissement, le mélange est hydrolysé dans de l'eau glacée. Le précipité obtenu est essoré, lavé à l'eau jusqu'à neutralité du filtrat et séché.
Recristalliser dans de l'éthanol.

Rendement : 60 %
Point de fusion : 189-191°C
Infrarouge :
v CO (thiocarbamate) : 1700 $cm^{-1}$
v CO (cétone) : 1660 $cm^{-1}$

Microanalyse élémentaire :

| | C % | H % |
|---|---|---|
| Calculé | 55,94 | 3,65 |
| Trouvé | 56,03 | 3,61 |

**EXEMPLE 16 : METHYL-3 ACETYL-6 BENZOTHIAZOLINONE**

Procéder de la même façon que dans l'exemple 15 en remplaçant la benzothiazolinone par la méthyl-3 benzothiazolinone et en maintenant l'agitation pendant 4 heures à 75 °C.
Recristalliser dans de l'éthanol absolu.

Rendement : 62 %
Point de fusion : 145-146 °C
Infrarouge :
v CO (thiocarbamate) : 1675 $cm^{-1}$
v CO (cétone) : 1660 $cm^{-1}$

Microanalyse élémentaire :

| | C % | H % |
|---|---|---|
| Calculé | 57,95 | 4,37 |
| Trouvé | 58,24 | 4,27 |

**EXEMPLE 17 : BROMOACETYL-6 BENZOTHIAZOLINONE**

Procéder de la même façon que dans l'exemple 15 en remplaçant le chlorure d'acide acétique par le chlorure d'acide bromoacétique et en maintenant l'agitation pendant une heure à 60 °C.

Recristalliser dans du dioxane.

Rendement : 65 %

Point de fusion : 240 °C

Infrarouge :

ν CO (thiocarbamate) : 1700 $cm^{-1}$

ν CO (cétone) : 1670 $cm^{-1}$

**EXEMPLE 18 : METHYL-3 BROMOACETYL-6 BENZOTHIAZOLINONE**

Procéder de la même façon que dans l'exemple 17 en remplaçant la benzothiazolinone par la méthyl-3 benzothiazolinone et en maintenant l'agitation pendant une heure à 65 °C.

Recristalliser dans de l'éthanol.

Rendement : 66 %

Point de fusion : 164 - 165 °C

Infrarouge :

ν CO (thiocarbamate) : 1675 $cm^{-1}$

ν CO (cétone) : 1655 $cm^{-1}$

**EXEMPLE 19 : (CHLORO-3 PROPIONYL)-6 BENZOTHIAZOLINONE**

Procéder de la même façon que dans l'exemple 15 en remplaçant le chlorure d'acide acétique par le chlorure d'acide chloro-3 propionique et en maintenant l'agitation pendant 2 heures à 80 °C.

Recristalliser dans de l'éthanol absolu.

Rendement : 55 %

Point de fusion : 174-175°C

Infrarouge :

ν CO (thiocarbamate et cétone) : entre 1680 $cm^{-1}$ et 1650 $cm^{-1}$

Microanalyse élémentaire :

| | C % | H % |
|---|---|---|
| Calculé | 49,69 | 3,33 |
| Trouvé | 49,51 | 3,42 |

**EXEMPLE 20 : METHYL-3 (CHLORO-3 PROPIONYL)-6 BENZOTHIAZOLINONE**

Procéder de la même façon que dans l'exemple 19 en remplaçant la benzothiazolinone par la méthyl-3 benzothiazolinone et en maintenant l'agitation pendant 2 heures à 85 °C.

Recristalliser dans de l'éthanol.

Rendement : 54 %

Point de fusion : 128-129 °C

Infrarouge :

ν CO (thiocarbamate et cétone) : 1660 $cm^{-1}$

Microanalyse élémentaire :

| | C % | H % |
|---|---|---|
| Calculé | 51,66 | 3,94 |
| Trouvé | 51,67 | 3,83 |

**EXEMPLE 21 : (CARBOXY-3 PROPIONYL)-6 BENZOTHIAZOLINONE**

Procéder de la même façon que dans l'exemple 15 en remplaçant le chlorure d'acide acétique par l'anhydride succinique et en maintenant l'agitation pendant 4 heures à 70 °C.

Recristalliser dans de l'éthanol.

Rendement : 55 %
Point de fusion : 242 °C
Infrarouge :
v CO (thiocarbamate) : 1680 $cm^{-1}$
v CO (cétone) : 1650 $cm^{-1}$

Microanalyse élémentaire :

| | C % | H % | S % |
|---|---|---|---|
| Calculé | 52,58 | 3,61 | 12,76 |
| Trouvé | 52,51 | 3,54 | 13,10 |

**EXEMPLE 22 : METHYL-3 (CARBOXY-3 PROPIONYL)-6 BENZOTHIAZOLINONE**

Procéder de la même façon que dans l'exemple 21 en remplaçant la benzothiazolinone par la méthyl-3 benzothiazolinone et en maintenant l'agitation pendant 3 heures à 75 °C.
Recristalliser dans de l'éthanol.
Rendement : 60 %
Point de fusion : 226-227 °C
Infrarouge :
v CO (thiocarbamate) : 1670 $cm^{-1}$
v CO (cétone) : 1650 $cm^{-1}$

Microanalyse élémentaire :

| | C % | H % |
|---|---|---|
| Calculé | 54,32 | 4,18 |
| Trouvé | 54,50 | 4,21 |

**EXEMPLE 23 : (CARBOXY-3 OXO-1 BUTEN-3-YL)-6 BENZOTHIAZOLINONE**

Procéder de la même façon que dans l'exemple 15 en remplaçant le chlorure d'acide acétique par l'anhydrique itaconique et en maintenant l'agitation pendant 6 heures à 75 °C.
Recristalliser dans de l'éthanol.
Rendement : 30 %
Point de fusion : 227-230 °C
Infrarouge :
v CO (thiocarbamate) : 1670 $cm^{-1}$
v CO (cétone) : 1635 $cm^{-1}$

Microanalyse élémentaire :

| | C % | H % | S % |
|---|---|---|---|
| Calculé | 54,74 | 3,44 | 12,16 |
| Trouvé | 54,81 | 3,42 | 12,17 |

**EXEMPLE 24 : METHYL-3 (CARBOXY-3 OXO-1 BUTEN-3-YL)-6 BENZOTHIAZOLINONE**

Procéder de la même façon que dans l'exemple 23 en remplaçant la benzothiazolinone par la méthyl-3 benzothiazolinone.

**EXEMPLE 25 : NICOTINOYL-6 BENZOTHIAZOLINONE**

Procéder de la même façon que dans l'exemple 15 en remplaçant le chlorure d'acide acétique par le chlorhydrate du chlorure d'acide nicotinique et en maintenant l'agitation pendant 30 heures à 100 °C.
Recristalliser dans de l'éthanol.
Rendement : 73 %
Point de fusion : 237-239 °C
Infrarouge :
ν CO (thiocarbamate) : 1720 $cm^{-1}$
ν CO (cétone) : 1635 $cm^{-1}$

Microanalyse élémentaire :

| | C % | H % | S % |
|---|---|---|---|
| Calculé | 60,92 | 3,14 | 12,51 |
| Trouvé | 61,12 | 3,13 | 12,36 |

**EXEMPLE 26 : METHYL-3 NICOTINOYL-6 BENZOTHIAZOLINONE**

Procéder de la même façon que dans l'exemple 25 en remplaçant la benzothiazolinone par la méthyl-3 benzothiazolinone et en maintenant l'agitation 30 heures à 90 °C.
Recristalliser dans de l'éthanol.
Rendement : 76 %
Point de fusion : 176-178 °C
Infrarouge :
ν CO (thiocarbamate) : 1670 $cm^{-1}$
ν CO (cétone) : 1640 $cm^{-1}$

Microanalyse élémentaire :

| | C % | H % | S % |
|---|---|---|---|
| Calculé | 62,20 | 3,73 | 11,86 |
| Trouvé | 62,19 | 3,67 | 11,55 |

**EXEMPLE 27 : METHYL-3 (HYDROXY-1 PHENYL-1 METHYL)-6 BENZOTHIAZOLINONE**

Dans une fiole de 250 $cm^3$, dissoudre dans 200 $cm^3$ de méthanol sous agitation magnétique, 0,02 mole de méthyl-3 benzoyl-6 benzothiazolinone préparée dans l'exemple 2. Ajouter très lentement et sous agitation 0,04 mole de borohydrure de sodium. Maintenir l'agitation 4 heures à température ambiante. Evaporer le milieu réactionnel au bain-marie sous vide. Reprendre le résidu par de l'eau, essorer le précipité formé et sécher.
Recristalliser dans du toluène.
Rendement : 92 %
Point de fusion : 129 - 130 °C
Infrarouge :ν CO (thiocarbamate) : 1635 $cm^{-1}$

**EXEMPLE 28 : (HYDROXY-1 PHENYL-1 METHYL)-6 BENZOTHIAZOLINONE**

Dans une fiole de 250 $cm^3$, dissoudre 0,02 mole de benzoyl-6 benzothiazolinone obtenue dans l'exemple 1 dans 80 $cm^3$ de solution aqueuse de soude à 30 %. Ajouter lentement et sous agitation 0,015 mole de borohydrure de sodium. Maintenir l'agitation 16 heures à température ambiante puis acidifier par de l'acide chlorhydrique dilué au demi. Essorer le précipité formé, laver à l'eau et sécher.
Recristalliser dans de l'acétonitrile.
Rendement : 91 %
Point de fusion : 159 - 160 °C

Infrarouge :ν CO (thiocarbamate) : 1645 $cm^{-1}$

**EXEMPLE 29 : (DICHLORO-2,6 BENZOYL)-6 BENZOTHIAZOLINONE**

Procéder comme dans l'exemple 1 en remplaçant l'acide benzoique par l'acide dichloro-2,6 benzoïque.
Solvant de cristallisation : méthanol
Point de fusion : > 260°C

**EXEMPLE 30 : p.ANISOYL-6 BENZOTHIAZOLINONE ou (METHOXY-4 BENZOYL)-6 BENZOTHIAZOLINONE**

Procéder comme dans l'exemple 1 en remplaçant l'acide benzoïque par l'acide p.anisique en maintenant l'agitation 4 heures à 120-123°C.
Solvant de cristallisation : dioxanne
Point de fusion :226-228°C

**EXEMPLE 31 : BENZOYL-6 BENZOTHIAZOLINONE, SEL DE DIETHANOLAMINE**

Dans une fiole de 250 $cm^3$ dissoudre 0,04 mole de benzoyl-6 benzothiazolinone dans 150 $cm^3$ de dioxanne.
Ajouter goutte à goutte 0,04 mole de diéthanolamine sous agitation magnétique. Agiter pendant deux heures. Essorer, sécher et recristalliser dans le dioxanne.
Point de fusion : 175°C

**EXEMPLE 32 : BENZOYL-6 BENZOTHIAZOLINONE, SEL DE SODIUM**

Dans un ballon de 250 $cm^3$ contenant 60 $cm^3$ d'éthanol, ajouter 0,04 atome gramme de sodium. Laisser reposer. Ajouter, sous agitation magnétique, 0,04 mole de benzoyl-6 benzothiazolinone. Laisser agiter une heure. Porter à sec. Chauffer le résidu à ébullition dans 80 $cm^3$ de dioxanne et essorer à chaud.
Point de fusion : < 260°C

**EXEMPLE 33 : PHENYL ACETYL-6 BENZOTHIAZOLINONE**

En procédant comme dans l'exemple 1, mais en remplaçant l'acide benzoïque par l'acide phénylacétique, on obtient le produit du titre.

**EXEMPLE 34 : p TOLUYL-6 BENZOTHIAZOLINONE**

En procédant comme dans l'exemple 1, mais en remplaçant l'acide benzoïque par l'acide para toluique, on obtient le produit du titre.

**EXEMPLE 35 : (TRIFLUOROMETHYL-4 BENZOYL)-6 BENZOTHIAZOLINONE**

En procédant comme dans l'exemple 1, mais en remplaçant l'acide benzoïque par l'acide trifluorométhyl-4 benzoïque, on obtient le produit du titre.

**EXEMPLE 36 : (TRIMETHOXY-3,4,5 BENZOYL)-6 BENZOTHIAZOLINONE**

Procéder de la même façon que dans l'exemple 1, en remplaçant l'acide benzoïque par l'acide triméthoxy-3,4,5 benzoïque.

**EXEMPLE 37 : (HYDROXY-4 BENZOYL)-6 BENZOTHIAZOLINONE**

Placer 0,02 mole de (méthoxy-4 benzoyl)-6 benzothiazolinone obtenue dans l'exemple 30 dans un mélange de 15 cc d'acide bromhydrique à 47% et 15 cc d'acide acétique. Porter à reflux sous agitation pendant 72 heures. Laisser refroidir, essorer, laver à l'eau et recristalliser dans l'alcool à 95°C.
Point de fusion : >265°C

**EXEMPLE 38 : [HYDROXY-1 (METHOXY-4 PHENYL)-1 METHYL]-6 BENZOTHIAZOLINONE**

Procéder comme dans l'exemple 28 en remplaçant la benzoyl-6 benzothiazolinone par la (méthoxy-4 benzoyl)-6 benzothiazolinone obtenue dans l'exemple 30. Maintenir l'agitation 5 jours.
Recristalliser dans l'éthanol dilué au demi
Point de fusion : 169-170°C

**EXEMPLE 39 : [HYDROXY-1 (CHLORO-4 PHENYL)-1 ETHYL]-6 BENZOTHIAZOLINONE**

Procéder comme dans l'exemple 38 en remplaçant la (méthoxy-4 benzoyl)-6 benzothiazolinone par la (chloro-4 benzoyl)-6 benzothiazolinone obtenue dans l'exemple 3.
Point de fusion : 154-155°C

**EXEMPLE 40 : [HYDROXY-1 -PENTYL]-6 BENZOTHIAZOLINONE**

Procéder comme dans l'exemple 28 en remplaçant la benzoyl-6 benzothiazolinone par la valéryl-6 benzothiazolinone obtenue dans l'exemple 9. Maintenir l'agitation 48 heures.
Recristalliser dans le toluène.
Point de fusion : 125-126°C

**ETUDE PHARMACOLOGIQUE DES DERIVES DE L'INVENTION**

**EXEMPLE 41 : ETUDE DE LA TOXICITE AIGUE**

La toxicité aiguë a été appréciée après administration orale à des lots de 8 souris (26 ± 2 grammes) d'une dose de 650 $mg.kg^{-1}$. Les animaux ont été observés à intervalles réguliers au cours de la première journée et quotidiennement pendant les 2 semaines suivant le traitement.

Il apparaît que les composés de l'invention sont totalement atoxiques. Aucun décès n'est observé après administration d'une dose de 650 $mg.kg^{-1}$. On ne constate pas de troubles après administration de cette dose.

**EXEMPLE 42 : ETUDE DE L'ACTIVITE ANALGESIQUE**

L'activité sur la douleur a été recherchée chez la souris (23-25 g) selon un protocole dérivé de la technique décrite par SIEGMUND (SIEGMUND E.A., R.A. CADMUS & GOLU, J. Pharm. Exp. Ther. 119, 1874, 1954). Les souris, réparties par randomisation en lots de 12 animaux, ont reçu le traitement par voie orale (excipient pour les témoins) 1 heure avant l'injection intra-péritonéale d'une solution hydroalcoolique de phényl-p-benzoquinone (Sigma) à 0,02 %. Les étirements sont dénombrés entre la 5ème et 10ème minute après l'injection.

Le pourcentage d'activité obtenu a été évalué pour chaque dose (% de diminution du nombre d'étirements chez les traités par rapport aux témoins). Une $ED_{50}$, dose entraînant une activité de 50 %, a été déterminée pour chaque produit.

Il est apparu que certains composés de l'invention possèdent une activité analgésique très intéressante. Ainsi, l'$ED_{50}$ du composé de l'exemple 1 est voisine de 2 $mg.kg^{-1}$.

A titre de comparaison l'administration d'une dose de 100 $mg.kg^{-1}$ des dérivés du brevet Fr 73.23280 provoquait un pourcentage d'analgésie - dans un test comparable - de l'ordre de 25 à 60 % et le composé du brevet Fr 80.20861 dont l'activité analgésique est la plus intéressante avait dans ce même test de Siegmund une $ED_{50}$ de 9 $mg.kg^{-1}$ soit environ 4 fois supérieure à celle du produit le plus intéressant de la présente invention.

**EXEMPLE 43 : ETUDE DE L'ACTIVITE NORMOLIPEMIANTE**

L'activité normolipémiante a été recherchée chez la souris (26-29 g) selon un protocole décrit par J.C. Fruchart et Coll. (Arthérosclérosis, 70 (1988), 107-114) permettant de mesurer le taux de cholestérol total ainsi que le taux d'HDL cholestérol sur des animaux ayant reçu une nourriture standard et hypercholestérolémique.

Il est apparu que les composés de l'invention possèdent une activité normolipémiante très intéressante. Ainsi, le composé décrit dans l'exemple 1 montre une diminution du taux de cholestérol total d'environ 30 % tout en augmentant le taux d'HDL cholestérol d'environ 30 %, ce qui est comparable à l'activité normolipémiante présentée par la fénofibrate utilisé comme témoin de référence.

**EXEMPLE 44 : ETUDE DE L'ACTIVITE ANTIAGREGANTE PLAQUETTAIRE**

Un plasma riche en plaquettes est préparé à partir de sang humain citraté, provenant de donneurs n'ayant pris aucun médicament pendant les dix jours précédant le prélèvement.
L'agrégation des plaquettes dans ce milieu plasmatique est étudiée par turbidimétrie en employant à des concentrations appropriées - un agoniste tel que l'ADP, l'adrénaline, le collagène, l'acide arachidonique. Les produits de l'invention sont ajoutés au plasma trois minutes avant l'agoniste.
Les produits de l'invention manifestent une activité antagoniste de l'agrégation plaquettaire significative.

**EXEMPLE 45 : COMPOSITION PHARMACEUTIQUE : COMPRIMES**

Comprimés dosés à 100 mg de benzoyl-6 benzothiazolinone
Formule de préparation pour 1000 comprimés.

| | |
|---|---|
| Benzoyl-6 benzothiazolinone | 100 g |
| Amidon de blé | 15 g |
| Amidon de maïs | 15 g |
| Lactose | 65 g |
| Stéarate de magnésium | 2 g |
| Silice | 1 g |
| Hydroxypropylcellulose | 2 g |

**Revendications**

**Revendications pour les Etats contractants suivants : AT, BE, CH, DE, DK, FR, GB, IT, LI, LU, NL, SE**

1. Composés de formule générale (I) :

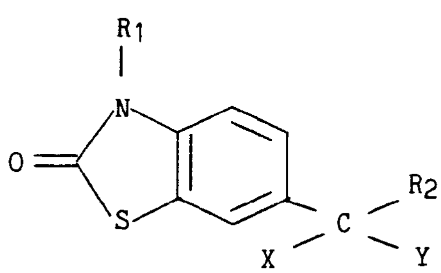

(I)

dans laquelle :

$R_1$ représente un atome d'hydrogène ou un groupement alkyle inférieur,

$R_2$ représente :

- . un groupement alkyle inférieur linéaire ou ramifié éventuellement substitué par un ou plusieurs atomes d'halogène ou par un ou plusieurs groupements alkoxy, hydroxy, phényle, acide carboxylique,
- . un groupement phényle éventuellement substitué par un ou plusieurs :
  - atomes d'halogène, ou
  - groupements alkyles inférieurs éventuellement substitués par un ou plusieurs atomes d'halogène, ou
  - groupements hydroxy ou alkoxy inférieurs,
- . un groupement alkényle inférieur linéaire ou ramifié éventuellement substitué par un groupement acide carboxylique, alkoxy, hydroxyle ou aryle,
- . un groupement thiényle-2, éventuellement substitué par un groupement alkyle inférieur linéaire ou ramifié,
- . un groupement furyle-2, éventuellement substitué par un groupement alkyle inférieur linéaire ou ramifié,
- . un groupement pyrrolyle-2, éventuellement substitué par un groupement alkyle inférieur linéaire ou ramifié,

. un groupement pyridyle, éventuellement substitué par un groupement alkyle inférieur linéaire ou ramifié,

X représente un atome d'hydrogène,

Y représente un groupement hydroxyle,

ou bien X et Y représentent ensemble un atome d'oxygène,

le terme inférieur indiquant que les groupements ainsi qualifiés comptent de 1 à 6 atomes de carbone, le terme inférieur qualifiant "alkényle" indique un groupement allant jusqu'à 6 atomes de carbone, leurs énantiomères, diastéréoisomères et épimères ainsi que lorsque $R_1$ représente un atome d'hydrogène et/ou $R_2$ comprend un groupement acide carboxylique, leurs sels d'addition à une base pharmaceutiquement acceptable, ainsi que lorsque $R_2$ comprend un groupement aminé, leurs sels d'addition à un acide pharmaceutiquement acceptable.

2. Composés selon la revendication 1, dans lesquels X et Y représentent ensemble un atome d'oxygène, leurs énantiomères, diastéréoisomères, et épimères ainsi que, lorsque $R_1$ représente un atome d'hydrogène et/ou $R_2$ comprend un groupement acide carboxylique, leurs sels d'addition à une base pharmaceutiquement acceptable, ainsi que lorsque $R_2$ comprend un groupement aminé, leurs sels d'addition à un acide pharmaceutiquement acceptable.

3. Composés selon la revendication 1, dans lesquels $R_1$ représente un atome d'hydrogène, leurs énantiomères, diastéréoisomères, et épimères ainsi que leurs sels d'addition à une base pharmaceutiquement acceptable, et lorsque $R_2$ comprend un groupement aminé, leurs sels d'addition à un acide pharmaceutiquement acceptable.

4. Composés selon la revendication 1, dans lesquels $R_1$ représente un groupement méthyle, leurs énantiomères, diastéréoisomères, et épimères ainsi que lorsque $R_2$ comprend un groupement acide carboxylique, leurs sels d'addition à une base pharmaceutiquement acceptable, et lorsque $R_2$ comprend un groupement aminé, leurs sels d'addition à un acide pharmaceutiquement acceptable.

5. Composés selon la revendication 1 dans lesquels $R_2$ représente un groupement phényle, leurs énantiomères, diastéréoisomères, épimères, ainsi que, lorsque $R_1$ représente un atome d'hydrogène, leurs sels d'addition à une base pharmaceutiquement acceptable.

6. Composés selon la revendication 1 dans lesquels $R_2$ représente un groupement alkyle, leurs énantiomères, diastéréoisomères, et épimères, atome d'hydrogène, ainsi que, lorsque $R_1$ représente un atome d'hydrogène, leurs sels d'addition à une base pharmaceutiquement acceptable.

7. Composés selon la revendication 1, qui est la benzoyl-6 benzothiazolinone, ainsi que ses sels d'addition à une base pharmaceutiquement acceptable.

8. Composés selon la revendication 1 dans lesquels $R_2$ représente un groupement phényle, ainsi que lorsque $R_1$ représente un atome d'hydrogène, leurs sels d'addition à une base pharmaceutiquement acceptable.

9. Composés selon la revendication 1 dans lesquels $R_2$ représente un groupement phényle substitué par un ou plusieurs atomes d'halogène, ainsi que, lorsque $R_1$ représente un atome d'hydrogène, leurs sels d'addition à une base pharmaceutiquement acceptable.

10. Composés selon la revendication 1 dans lesquels $R_2$ représente un groupement thiényle, ainsi que lorsque $R_1$ représente un atome d'hydrogène, leurs sels d'addition à une base pharmaceutiquement acceptable.

11. Composés selon la revendication 1 dans lesquels $R_2$ représente un groupement alkyle inférieur ou phényle substitué par un groupement hydroxyle, leurs énantiomères, diastéréoisomères, épimères ainsi que lorsque $R_1$ représente un atome d'hydrogène, leurs sels d'addition à une base pharmaceutiquement acceptable.

12. Composés selon la revendication 1 dans lesquels $R_2$ représente un groupement alkyle inférieur substitué par un atome d'halogène, leurs énantiomères, diastéréoisomères, épimères ainsi que, lorsque $R_1$ représente un atome d'hydrogène, leurs sels d'addition à une base pharmaceutiquement acceptable.

13. Composés selon la revendication 1 dans lesquels $R_2$ représente un groupement alkyle inférieur substitué par un groupement acide carboxylique, leurs énantiomères, diastéréoisomères et épimères ainsi que leurs

sels d'addition à une base pharmaceutiquement acceptable.

**14.** Composés selon la revendication 1 dans lesquels $R_2$ représente un groupement alcényle inférieur substitué par un groupement acide carboxylique, leurs énantiomères, diastéréoisomères, et épimères ainsi que leurs sels d'addition à une base pharmaceutiquement acceptable.

**15.** Composés selon la revendication 1 dans lesquels $R_2$ représente un groupement pyridyle leurs énantiomères, diastéréoisomères et épimères ainsi que leurs sels d'addition à un acide pharmaceutiquement acceptable, et lorsque $R_1$ représente un atome d'hydrogène, leurs sels d'addition à une base pharmaceutiquement acceptable.

**16.** Composés selon la revendication 1 dans lesquels X représente un atome d'hydrogène, Y représente un groupement hydroxyle, leurs énantiomères, diastéréoisomères et épimères ainsi que, lorsque $R_1$ représente un atome d'hydrogène, leurs sels d'addition à une base pharmaceutiquement acceptable et lorsque $R_2$ comprend un groupement aminé leurs sels d'addition à un acide pharmaceutiquement acceptable.

**17.** Composés selon la revendication 1 dans lesquels $R_2$ représente un groupement phényle alkyle inférieur leurs énantiomères, diastéréoisomères et épimères ainsi que lorsque $R_1$ représente un atome d'hydrogène leurs sels d'addition à une base pharmaceutiquement acceptable.

**18.** Composés selon la revendication 1 dans lesquels $R_2$ représente un groupement phényle substitué par un ou plusieurs groupements choisis parmi alkyle inférieur, alkoxy inférieur, hydroxy, trifluorométhyle leurs isomères, diastéréoisomères, épimères, ainsi que lorsque $R_1$ représente un atome d'hydrogène, leurs sels d'addition à une base pharmaceutiquement acceptable.

**19.** Procédé de préparation des composés de formule générale (I), caractérisé en ce que l'on utilise comme matière première un dérivé de formule (II) :

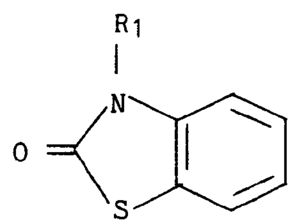

(II)

dans laquelle $R_1$ a la même signification que dans la formule (I),
que l'on soumet, en fonction de la nature de $R_2$,

* ou bien à l'action d'un chlorure d'acide de formule (III) :

$$R_2 - CO - Cl \qquad (III)$$

dans laquelle $R_2$ a la même signification que dans la formule (I),
ou bien de l'anhydride d'acide ou de la lactone correspondant,
préférentiellement en présence de chlorure d'aluminium dans le diméthylformamide,
pour obtenir un dérivé de formule (I/a) :

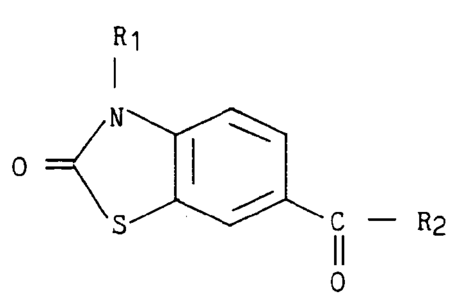

(I/a)

cas particulier des dérivés de formule (I),
dans laquelle :

$R_1$ et $R_2$ ont la même signification que dans la formule (I),

X et Y représentent ensemble un atome d'oxygène,

* ou bien à l'action d'un acide de formule (V) :

$$R_2 - CO_2H \qquad (V)$$

dans laquelle $R_2$ a la même signification que dans la formule (I), ou du chlorure d'acide ou de l'anhydride d'acide ou de la lactone correspondant en présence d'acide polyphosphorique pour obtenir un dérivé de formule (I/a) :

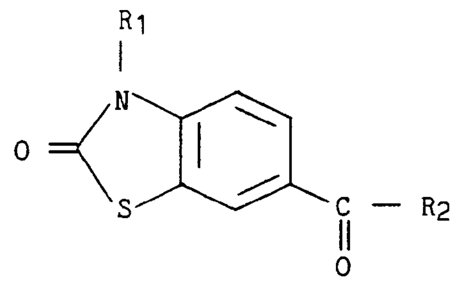

(I/a)

cas particulier des dérivés de formule (I), dans laquelle :

$R_1$ et $R_2$ ont la même signification que dans la formule (I),

X et Y représentent ensemble un atome d'oxygène,

dérivé de formule (I/a) que l'on purifie éventuellement par une technique classique de purification, et que, l'on peut, si on le désire, dans les cas où $R_1$ représente un atome d'hydrogène, où dans les cas où $R_2$ comprend un groupement acide carboxylique ou un groupement amine, salifier respectivement par une base ou un acide pharmaceutiquement acceptable,

et qui, lorsque $R_2$ représente un groupement aryle substitué par un ou plusieurs groupements alkoxy inférieurs peut être soumis, si on le désire, à l'action d'un acide fort, pour conduire à un dérivé de formule de (I/z) :

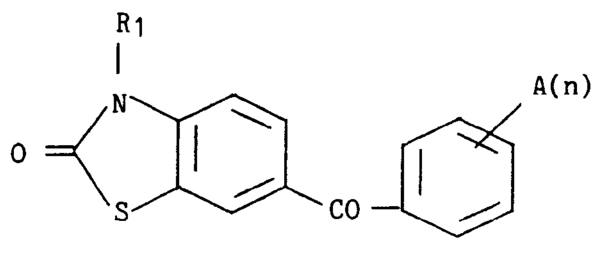

(I/z)

dans laquelle $R_1$ a la même signification que dans la formule (I), Areprésente un groupement hydroxy et n est un nombre entier compris entre 1 et 5,

la position du (des) groupement(s) hydroxy étant identique à celle de (des) groupement(s) alkoxy inférieur du produit de formule (I) soumis à l'action de l'acide,

produit de formule (I/z) que l'on purifie éventuellement par une technique classique et que, l'on peut, dans les cas où $R_1$ représente un atome d'hydrogène, si on le désire, salifier respectivement par une base pharmaceutiquement acceptable,

dérivé de formule (I/a) ou de formule (I/z) qui, si on le souhaite, peut être soumis à l'action d'un agent d'hydrogénation choisi parmi un hydrure mixte de métal alcalin comme par exemple le borohydrure de sodium, préférentiellement en présence d'alcool aliphatique inférieur, pour conduire à un dérivé de formule (I/b) :

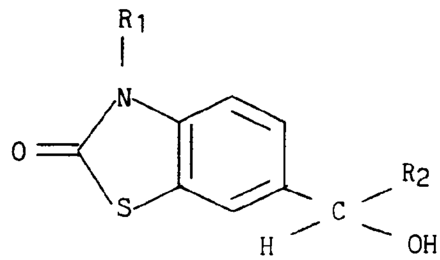

(I/b)

cas particulier des dérivés de formule (I) dans laquelle :

$R_1$ et $R_2$ ont la même signification que dans la formule (I),
X représente un atome d'hydrogène,
Y représente un groupement hydroxyle,

dont on sépare, si on le souhaite, les isomères, par une technique classique de séparation et que l'on purifie éventuellement par une technique classique de purification et que - dans le cas où $R_1$ représente un atome d'hydrogène ou dans le cas où $R_2$ comprend un groupement acide carboxylique l'on peut salifier par une base pharmaceutiquement acceptable ou encore que l'on peut dans les cas où $R_2$ comprend un groupement aminé, salifier par un acide pharmaceutiquement acceptable.

**20.** Procédé de préparation selon la revendication 19 de composés de formule (I/z) :

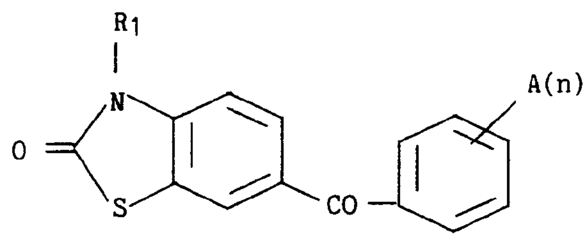

(I/z)

cas particulier des dérivés de formule (I) dans lequel R1 a la même signification que dans la formule (I), A représente un groupement hydroxy et n est un nombre entier compris entre 1 et 5 caractérisé en ce que l'on soumet un dérivé de formule (I) selon la revendication 1 dans lequel le groupement R2 signifie un radical phényle substitué par 1 à 5 groupements alkoxy inférieur à l'action d'un acide fort,
pour conduire à un dérivé de formule (I/z), la position des groupements hydroxy du dérivé ainsi obtenu étant identique à celle des groupements alkoxy inférieur du produit de formule (I) soumis à l'acide fort, produit de formule (I/z) que l'on purifie éventuellement par une technique classique et que, l'on peut, dans le cas où $R_1$ représente un atome d'hydrogène, si on le désire, salifier par une base pharmaceutiquement acceptable.

**21.** Composition pharmaceutique contenant comme principe actif au moins un composé selon l'une des revendications 1 à 18 en combinaison avec un ou plusieurs excipients ou véhicules inertes, non toxiques, pharmaceutiquement acceptables.

**22.** Composition pharmaceutique selon la revendication 21 contenant au moins un principe actif selon l'une des revendications 1 à 18 utilisables comme analgésique ou comme normolipémiant et dans le traitement des maladies artérielles ischémiques.

**Revendications pour les Etats contractants suivants : ES, GR**

**1.** Procédé de préparation de composés de formule générale (I) :

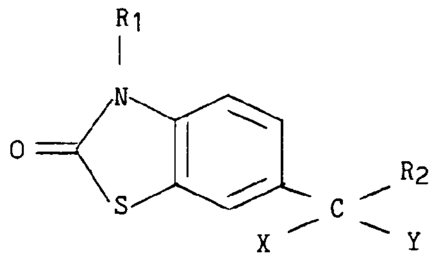

(I)

dans laquelle :

$R_1$ représente un atome d'hydrogène ou un groupement alkyle inférieur,

$R_2$ représente :

. un groupement alkyle inférieur linéaire ou ramifié éventuellement substitué par un ou plusieurs atomes d'halogène ou par un ou plusieurs groupements alkoxy, hydroxy, phényle, acide carboxylique,

. un groupement phényle éventuellement substitué par un ou plusieurs :
- atomes d'halogène, ou
- groupements alkyles inférieurs éventuellement substitués par un ou plusieurs atomes d'halogène, ou
- groupements hydroxy ou alkoxy inférieurs,

. un groupement alkényle inférieur linéaire ou ramifié éventuellement substitué par un groupement acide carboxylique, alkoxy, hydroxyle ou aryle,

. un groupement thiényle-2, éventuellement substitué par un groupement alkyle inférieur linéaire ou ramifié,

. un groupement furyle-2, éventuellement substitué par un groupement alkyle inférieur linéaire ou ramifié,

. un groupement pyrrolyle-2, éventuellement substitué par un groupement alkyle inférieur linéaire ou ramifié,

. un groupement pyridinyle, éventuellement substitué par un groupement alkyle inférieur linéaire ou ramifié,

X représente un atome d'hydrogène,

Y représente un groupement hydroxyle,

ou bien X et Y représentent ensemble un atome d'oxygène,

le terme inférieur indiquant que les groupements ainsi qualifiés comptent de 1 à 6 atomes de carbone,

le terme inférieur qualifiant "alkényle" indique un groupement allant jusqu'à 6 atomes de carbone,

leurs énantiomères, diastéréoisomères et épimères ainsi que lorsque $R_1$ représente un atome d'hydrogène et/ou $R_2$ comprend un groupement acide carboxylique, leurs sels d'addition à une base pharmaceutiquement acceptable, ainsi que lorsque $R_2$ comprend un groupement aminé, leurs sels d'addition à un acide pharmaceutiquement acceptable,

caractérisé en ce que l'on utilise comme matière première un dérivé de formule (II) :

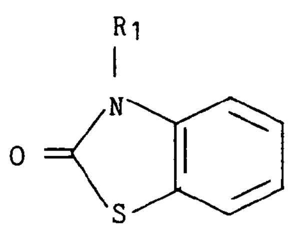

(II)

dans laquelle $R_1$ a la même signification que dans la formule (I),

que l'on soumet, en fonction de la nature de $R_2$,

* ou bien à l'action d'un chlorure d'acide de formule (III) :

$$R_2 - CO - Cl \qquad (III)$$

dans laquelle $R_2$ a la même signification que dans la formule (I),

ou bien de l'anhydride d'acide ou de la lactone correspondant, préférentiellement en présence de chlorure d'aluminium dans le diméthylformamide,

pour obtenir un dérivé de formule (I/a) :

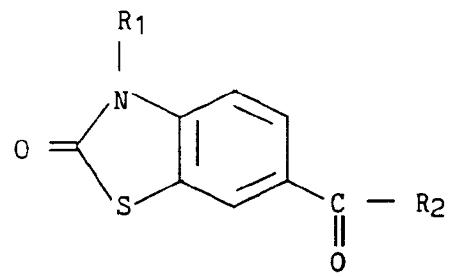

(I/a)

cas particulier des dérivés de formule (I),
dans laquelle :

$R_1$ et $R_2$ ont la même signification que dans la formule (I),
X et Y représentent ensemble un atome d'oxygène,

* ou bien à l'action d'un acide de formule (V) :

**$R_2$ - $CO_2H$ (V)**

dans laquelle $R_2$ a la même signification que dans la formule (I),
ou du chlorure d'acide ou de l'anhydride d'acide ou de la lactone correspondant en présence d'acide polyphosphorique
pour obtenir un dérivé de formule (I/a) :

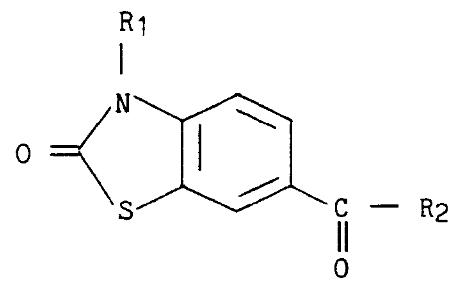

(I/a)

cas particulier des dérivés de formule (I),
dans laquelle :

$R_1$ et $R_2$ ont la même signification que dans la formule (I),
X et Y représentent ensemble un atome d'oxygène,

dérivé de formule (I/a) que l'on purifie éventuellement par une technique classique de purification, et que, l'on peut, si on le désire, dans les cas où $R_1$ représente un atome d'hydrogène, où dans les cas où $R_2$ comprend un groupement acide carboxylique ou un groupement amine, salifier respectivement par une base ou un acide pharmaceutiquement acceptable,
et qui, lorsque $R_2$ représente un groupement aryle substitué par un ou plusieurs groupements alkoxy inférieurs peut être soumis, si on le désire, à l'action d'un acide fort, pour conduire à un dérivé de forumule de (I/z) :

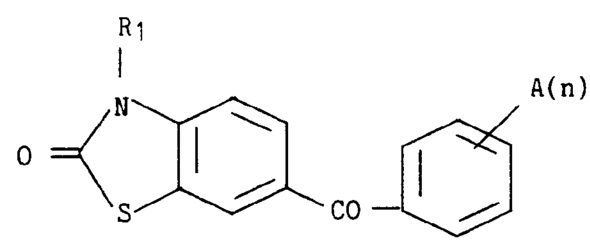

(I/z)

dans laquelle $R_1$ a la même signification que dans la formule (I), A représente un groupement hydroxy et n est un nombre entier compris entre 1 et 5,
la position du (des) groupement(s) hydroxy étant identique à celle de (des) groupement(s) alkoxy inférieur du produit de formule (I) soumis à l'action de l'acide,
produit de formule (I/z) que l'on purifie éventuellement par une technique classique et que, l'on peut, dans les cas où $R_1$ représente un atome d'hydrogène, si on le désire, salifier respectivement par une

base pharmaceutiquement acceptable,
dérivé de formule (I/a) ou de formule (I/z) qui, si on le souhaite, peut être soumis à l'action d'un agent d'hydrogénation choisi parmi un hydrure mixte de métal alcalin comme par exemple le borohydrure de sodium, préférentiellement en présence d'alcool aliphatique inférieur, pour conduire à un dérivé de formule (I/b) :

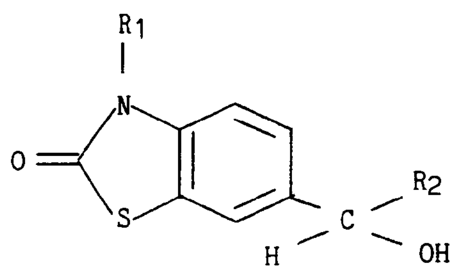

(I/b)

cas particulier des dérivés de formule (I) dans laquelle :

$R_1$ et $R_2$ ont la même signification que dans la formule (I),
X représente un atome d'hydrogène,
Y représente un groupement hydroxyle,

dont on sépare, si on le souhaite, les isomères, par une technique classique de séparation et que l'on purifie éventuellement par une technique classique de purification et que - dans le cas où $R_1$ représente un atome d'hydrogène ou dans le cas où $R_2$ comprend un groupement acide carboxylique l'on peut salifier par une base pharmaceutiquement acceptable ou encore que l'on peut dans les cas où $R_2$ comprend un groupement aminé, salifier par un acide pharmaceutiquement acceptable.

2. Procédé de préparation selon la revendication 1 de composés de formule (I/z) :

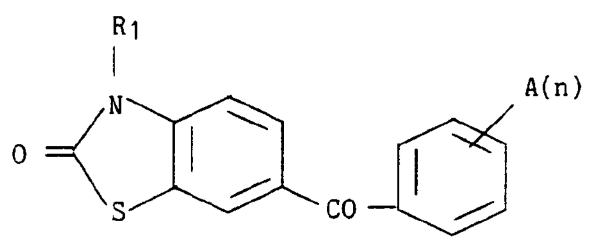

(I/z)

cas particulier des dérivés de formule (I) dans lequel R1 a la même signification que dans la formule (I), A représente un groupement hydroxy et n est un nombre entier compris entre 1 et 5 caractérisé en ce que l'on soumet un dérivé de formule (I) selon la revendication 1 dans lequel le groupement R2 signifie un radical phényle substitué par 1 à 5 groupements alkoxy inférieur à l'action d'un acide fort,
pour conduire à un dérivé de formule (I/z), la position des groupements hydroxy du dérivé ainsi obtenu étant identique à celle des groupements alkoxy inférieur du produit de formule (I) soumis à l'acide fort, produit de formule (I/z) que l'on purifie éventuellement par une technique classique et que, l'on peut, dans le cas où $R_1$ représente un atome d'hydrogène, si on le désire, salifier par une base pharmaceutiquement acceptable.

3. Procédé de préparation selon la revendication 1, de composés de formule (I) dans lesquels X et Y représentent ensemble un atome d'oxygène, leurs énantiomères, diastéréoisomères, et épimères ainsi que, lorsque $R_1$ représente un atome d'hydrogène et/ou $R_2$ comprend un groupement acide carboxylique, leurs sels d'addition à une base pharmaceutiquement acceptable, ainsi que lorsque $R_2$ comprend un groupement aminé, leurs sels d'addition à un acide pharmaceutiquement acceptable.

4. Procédé de préparation selon la revendication 1, de composés de formule (I) dans lesquels $R_1$ représente un atome d'hydrogène, leurs énantiomères, diastéréoisomères, et épimères ainsi que leurs sels d'addition à une base pharmaceutiquement acceptable, et lorsque $R_2$ comprend un groupement aminé, leurs sels d'addition à un acide pharmaceutiquement acceptable.

5. Procédé de préparation selon la revendication 1, de composés de formule (I), dans lesquels $R_1$ représente un groupement méthyle, leurs énantiomères, diastéréoisomères, et épimères ainsi que, lorsque $R_2$ comprend un groupement acide carboxylique, leurs sels d'addition à une base pharmaceutiquement acceptable, et lorsque $R_2$ comprend un groupement aminé, leurs sels d'addition à un acide pharmaceutiquement acceptable.

6. Procédé de préparation selon la revendication 1, de composés de formule (I) dans lesquels $R_2$ représente un groupement phényle, leurs énantiomères, diastéréoisomères, épimères, ainsi que, lorsque $R_1$ représente un atome d'hydrogène, leurs sels d'addition à une base pharmaceutiquement acceptable.

7. Procédé de préparation selon la revendication 1, de composés de formule (I) dans lesquels $R_2$ représente un groupement alkyle, leurs énantiomères, diastéréoisomères, et épimères, atome d'hydrogène, ainsi que, lorsque R1 représente un atome d'hydrogène, leurs sels d'addition à une base pharmaceutiquement acceptable.

8. Procédé de préparation selon la revendication 1, du composé de formule (I) qui est la benzoyl-6 benzothiazolinone, ainsi que ses sels d'addition à une base pharmaceutiquement acceptable.

9. Procédé de préparation selon la revendication 1, de composés de formule (I) dans lesquels $R_2$ représente un groupement phényle, ainsi que lorsque $R_1$ représente un atome d'hydrogène, leurs sels d'addition à une base pharmaceutiquement acceptable.

10. Procédé de préparation selon la revendication 1, de composés de formule (I) dans lesquels $R_2$ représente un groupement phényle substitué par un ou plusieurs atomes d'halogène, ainsi que, lorsque $R_1$ représente un atome d'hydrogène, leurs sels d'addition à une base pharmaceutiquement acceptable.

11. Procédé de préparation selon la revendication 1 de composés de formule (I) dans lesquels $R_2$ représente un groupement thiényle, ainsi que lorsque $R_1$ représente un atome d'hydrogène, leurs sels d'addition à une base pharmaceutiquement acceptable.

12. Procédé de préparation selon la revendication 1 de composés de formule (I) dans lesquels $R_2$ représente un groupement alkyle inférieur ou phényle substitué par un groupement hydroxyle, leurs énantiomères, diastéréoisomères, épimères ainsi que lorsque $R_1$ représente un atome d'hydrogène, leurs sels d'addition à une base pharmaceutiquement acceptable.

13. Procédé de préparation selon la revendication 1 de composés de formule (I) dans lesquels $R_2$ représente un groupement alkyle inférieur substitué par un atome d'halogène, leurs énantiomères, diastéréoisomères, épimères ainsi que, lorsque $R_1$ représente un atome d'hydrogène, leurs sels d'addition à une base pharmaceutiquement acceptable.

14. Procédé de préparation selon la revendication 1 de composés de formule (I) dans lesquels $R_2$ représente un groupement alkyle inférieur substitué par un groupement acide carboxylique, leurs énantiomères, diastéréoisomères et épimères ainsi que leurs sels d'addition à une base pharmaceutiquement acceptable.

15. Procédé de préparation selon la revendication 1 de composés de formule (I) dans lesquels $R_2$ représente un groupement alcényle inférieur substitué par un groupement acide carboxylique, leurs énantiomères, diastéréoisomères, et épimères ainsi que leurs sels d'addition à une base pharmaceutiquement acceptable.

16. Procédé de préparation selon la revendication 1 de composés de formule (I) dans lesquels $R_2$ représente un groupement pyridyle leurs énantiomères, diastéréoisomères et épimères ainsi que leurs sels d'addition à un acide pharmaceutiquement acceptable, et lorsque $R_1$ représente un atome d'hydrogène, leurs sels d'addition à une base pharmaceutiquement acceptable.

17. Procédé de préparation selon la revendication 1 de composés de formule (I) dans lesquels X représente un atome d'hydrogène, Y représente un groupement hydroxyle, leurs énantiomères, diastéréoisomères et épimères ainsi que, lorsque $R_1$ représente un atome d'hydrogène, leurs sels d'addition à une base pharmaceutiquement acceptable et lorsque $R_2$ comprend un groupement aminé, leurs sels d'addition à un acide pharmaceutiquement acceptable.

18. Procédé de préparation selon la revendication 1 de composés de formule (I) dans lesquels $R_2$ représente un groupement phényle alkyle inférieur leurs énantiomères, diastéréoisomères et épimères ainsi que lorsque $R_1$ représente un atome d'hydrogène leurs sels d'addition à une base pharmaceutiquement acceptable.

19. Procédé de préparation selon l'une des revendications 1 ou 2 de composés de formule (I) dans lesquels $R_2$ représente un groupement phényle substitué par un ou plusieurs groupements choisis parmi alkyle inférieur, alkoxy inférieur, hydroxy, trifluorométhyle leurs isomères, diastéréoisomères, épimères, ainsi que lorsque $R_1$ représente un atome d'hydrogène, leurs sels d'addition à une base pharmaceutiquement acceptable.

20. Procédé de préparation de compositions pharmaceutiques contenant comme principe actif au moins un composé obtenu selon l'une des revendications 1 à 19 en combinaison avec un ou plusieurs excipients ou véhicules inertes, non toxiques, pharmaceutiquement acceptables.

## Claims

### Claims for the following Contracting States : AT, BE, CH, DE, DK, FR, GB, IT, LI, LU, NL, SE

1. Compounds of the general formula (I):

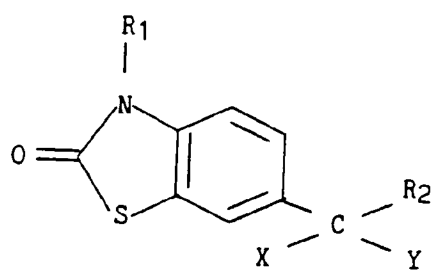

(I)

in which:

$R_1$ represents a hydrogen atom or a lower alkyl grouping,

$R_2$ represents:

. a linear or branched lower alkyl grouping optionally substituted by one or more halogen atoms or by one or more alkoxy, hydroxy, phenyl, carboxylic acid groupings,

. a phenyl grouping optionally substituted by one or more:

- halogen atoms, or
- lower alkyl groupings optionally substituted by one or more halogen atoms, or
- hydroxy or lower alkoxy groupings,

. a linear or branched lower alkenyl grouping optionally substituted by a carboxylic acid, alkoxy, hydroxy or aryl grouping,

. a 2-thienyl grouping optionally substituted by a linear or branched lower alkyl grouping,

. a 2-furyl grouping optionally substituted by a linear or branched lower alkyl grouping,

. a 2-pyrrolyl grouping optionally substituted by a linear or branched lower alkyl grouping, or

. a pyridyl grouping optionally substituted by a linear or branched lower alkyl grouping,

X represents a hydrogen atom,

Y represents a hydroxy grouping,

or X and Y together represent an oxygen atom,

the term "lower" indicating that the groupings so qualified contain from 1 to 6 carbon atoms,

the term "lower" qualifying alkenyl indicating a grouping having up to 6 carbon atoms,

their enantiomers, diastereoisomers and epimers and also, when $R_1$ represents a hydrogen atom and/or $R_2$ contains a carboxylic acid grouping, their addition salts with a pharmaceutically acceptable base, and also, when $R_2$ contains an amino grouping, their addition salts with a pharmaceutically acceptable acid.

2. Compounds according to claim 1 in which X and Y together represent an oxygen atom, their enantiomers, diastereoisomers and epimers and also, when $R_1$ represents a hydrogen atom and/or $R_2$ contains a car-

boxylic acid grouping, their addition salts with a pharmaceutically acceptable base and also, when $R_2$ contains an amino grouping, their addition salts with a pharmaceutically acceptable acid.

3. Compounds according to claim 1 in which $R_1$ represents a hydrogen atom, their enantiomers, diastereoisomers and epimers and also their addition salts with a pharmaceutically acceptable base and, when $R_2$ contains an amino grouping, their addition salts with a pharmaceutically acceptable acid.

4. Compounds according to claim 1 in which $R_1$ represents a methyl grouping, their enantiomers, diastereoisomers and epimers and also, when $R_2$ contains a carboxylic acid grouping, their addition salts with a pharmaceutically acceptable base and, when $R_2$ contains an amino grouping, their addition salts with a pharmaceutically acceptable acid.

5. Compounds according to claim 1 in which $R_2$ represents a phenyl grouping, their enantiomers, diastereoisomers and epimers and also, when $R_1$ represents a hydrogen atom, their addition salts with a pharmaceutically acceptable base.

6. Compounds according to claim 1 in which $R_2$ represents an alkyl grouping, their enantiomers, diastereoisomers and epimers, hydrogen atom, and also, when $R_1$ represents a hydrogen atom, their addition salts with a pharmaceutically acceptable base.

7. Compound according to claim 1 which is 6-benzoyl-benzothiazolinone, and also its addition salts with a pharmaceutically acceptable base.

8. Compounds according to claim 1 in which $R_2$ represents a phenyl grouping, and also, when $R_1$ represents a hydrogen atom, their addition salts with a pharmaceutically acceptable base.

9. Compounds according to claim 1 in which $R_2$ represents a phenyl grouping substituted by one or more halogen atoms, and also, when $R_1$ represents a hydrogen atom, their addition salts with a pharmaceutically acceptable base.

10. Compounds according to claim 1 in which $R_2$ represents a thienyl grouping, and also, when $R_1$ represents a hydrogen atom, their addition salts with a pharmaceutically acceptable base.

11. Compounds according to claim 1 in which $R_2$ represents a lower alkyl or phenyl grouping substituted by a hydroxy grouping, their enantiomers, diastereoisomers and epimers and also, when $R_1$ represents a hydrogen atom, their addition salts with a pharmaceutically acceptable base.

12. Compounds according to claim 1 in which $R_2$ represents a lower alkyl grouping substituted by a halogen atom, their enantiomers, diastereoisomers and epimers and also, when $R_1$ represents a hydrogen atom, their addition salts with a pharmaceutically acceptable base.

13. Compounds according to claim 1 in which $R_2$ represents a lower alkyl grouping substituted by a carboxylic acid grouping, their enantiomers, diastereoisomers and epimers and also their addition salts with a pharmaceutically acceptable base.

14. Compounds according to claim 1 in which $R_2$ represents a lower alkenyl grouping substituted by a carboxylic acid grouping, their enantiomers, diastereoisomers and epimers and also their addition salts with a pharmaceutically acceptable base.

15. Compounds according to claim 1 in which $R_2$ represents a pyridyl grouping, their enantiomers, diastereoisomers and epimers and also their addition salts with a pharmaceutically acceptable acid and, when $R_1$ represents a hydrogen atom, their addition salts with a pharmaceutically acceptable base.

16. Compounds according to claim 1 in which X represents a hydrogen atom, Y represents a hydroxy grouping, their enantiomers, diastereoisomers and epimers and also, when $R_1$ represents a hydrogen atom, their addition salts with a pharmaceutically acceptable base and, when $R_2$ contains an amino grouping, their addition salts with a pharmaceutically acceptable acid.

17. Compounds according to claim 1 in which $R_2$ represents a lower alkylphenyl grouping, their enantiomers, diastereoisomers and epimers and also, when $R_1$ represents a hydrogen atom, their addition salts with a

pharmaceutically acceptable base.

**18.** Compounds according to claim 1 in which $R_2$ represents a phenyl grouping substituted by one or more groupings selected from lower alkyl, lower alkoxy, hydroxy and trifluoromethyl, their isomers, diastereoisomers and epimers and also, when $R_1$ represents a hydrogen atom, their addition salts with a pharmaceutically acceptable base.

**19.** Process for the preparation of the compounds of the general formula (I), characterised in that there is used as starting material a compound of formula (II):

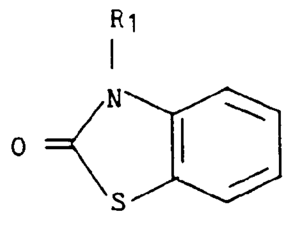

(II)

in which $R_1$ is as defined in formula (I),
which is subjected, depending on the nature of $R_2$,

* either to the action of an acid chloride of formula (III):

$$R_2 - CO - Cl \qquad (III)$$

in which $R_2$ is as defined in formula (I),
or the corresponding acid anhydride or lactone,
preferably in the presence of aluminium chloride in dimethylformamide,
to obtain a compound of formula (I/a):

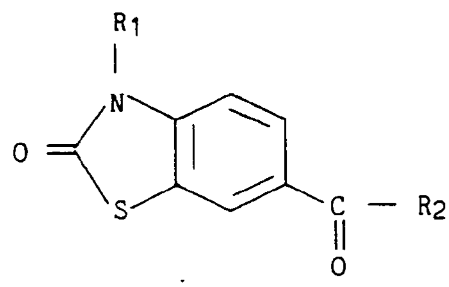

(I/a)

a particular case of the compounds of formula (I),
in which:
$R_1$ and $R_2$ are as defined in formula (I), and
X and Y together represent an oxygen atom,

* or to the action of an acid of formula (V):

$$R_2 - CO_2H \qquad (V)$$

in which $R_2$ is as defined in formula (I),
or the corresponding acid chloride or acid anhydride or lactone in the presence of polyphosphoric acid
to obtain a compound of formula (I/a):

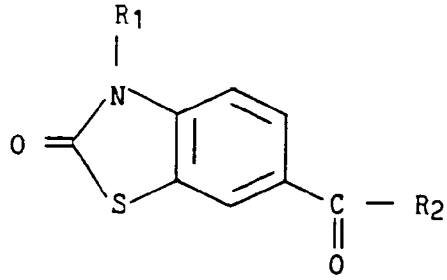

(I/a)

a particular case of the compounds of formula (I), in which: $R_1$ and $R_2$ are as defined in formula (I), and X and Y together represent an oxygen atom, which compound of formula (I/a) is optionally purified by a conventional purification technique and, if desired, in the cases where $R_1$ represents a hydrogen atom, or in the cases where $R_2$ contains a carboxylic acid grouping or an amino grouping, may be converted into a salt by means of a pharmaceutically acceptable base or acid, respectively, and which, when $R_2$ represents an aryl grouping substituted by one or more lower alkoxy groupings, may be subjected, if desired, to the action of a strong acid to yield a compound of formula (I/z):

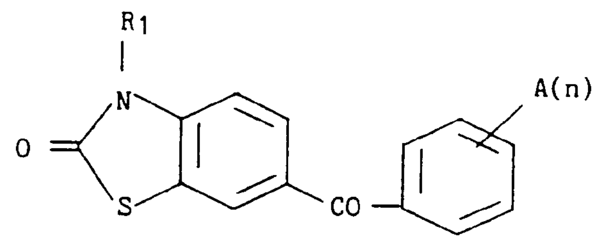

(I/z)

in which $R_1$ is as defined in formula (I), A represents a hydroxy grouping and n is an integer from 1 to 5, the position of the hydroxy grouping(s) being identical with that of the lower alkoxy grouping(s) of the product of formula (I) that was subjected to the action of the acid, which product of formula (I/z) is optionally purified by a conventional technique and, if desired, in the cases where $R_1$ represents a hydrogen atom, may be converted into a salt by means of a pharmaceutically acceptable base, which compound of formula (I/a) or of formula (I/z) may, if desired, be subjected to the action of a hydrogenation agent selected from mixed alkali metal hydrides, such as, for example, sodium borohydride, preferably in the presence of a lower aliphatic alcohol, to yield a compound of formula (I/b):

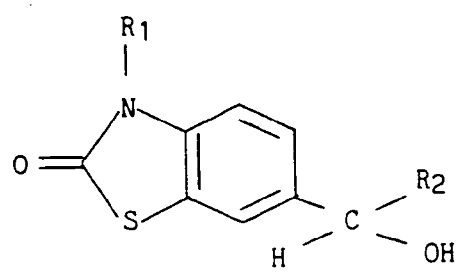

(I/b)

a particular case of the compounds of formula (I) in which: $R_1$ and $R_2$ are as defined in formula (I), X represents a hydrogen atom, and Y represents a hydroxy grouping, the isomers of which are, if desired, separated by a conventional separation technique and which is optionally purified by a conventional purification technique and which, in the case where $R_1$ represents a hydrogen atom or in the case where $R_2$ contains a carboxylic acid grouping, may be converted into a salt by means of a pharmaceutically acceptable base or which, in the cases where $R_2$ contains an amino grouping, may be converted into a salt by means of a pharmaceutically acceptable acid.

**20.** Process for the preparation according to claim 19 of compounds of formula (I/z):

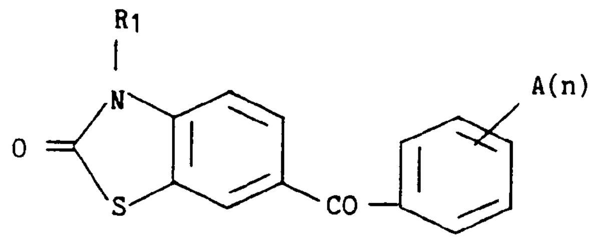

(I/z)

a particular case of the compounds of formula (I) in which $R_1$ is as defined in formula (I), A represents a hydroxy grouping and n is an integer from 1 to 5, characterised in that a compound of formula (I) according to claim 1 in which the $R_2$ grouping represents a phenyl radical substituted by from 1 to 5 lower alkoxy groupings is subjected to the action of a strong acid,
to yield a compound of formula (I/z), the position of the hydroxy groupings of the compound so obtained being identical with that of the lower alkoxy groupings of the product of formula (I) that was subjected to the strong acid,
which product of formula (I/z) is optionally purified by a conventional technique and, in the case where $R_1$ represents a hydrogen atom, may, if desired, be converted into a salt by means of a pharmaceutically acceptable base.

**21.** Pharmaceutical composition containing as active ingredient at least one compound according to any one of claims 1 to 18 in combination with one or more pharmaceutically acceptable, inert, non-toxic excipients or carriers.

**22.** Pharmaceutical composition according to claim 21 containing at least one active ingredient according to any one of claims 1 to 18 for use as an analgesic or as a normolipaemic agent and in the treatment of ischaemic arterial disorders.

**Claims for the following Contracting States : ES, GR**

**1.** Process for the preparation of compounds of the general formula (I):

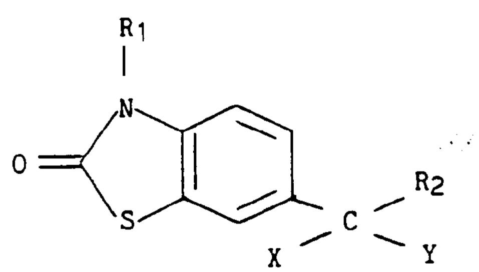

(I)

in which:

$R_1$ represents a hydrogen atom or a lower alkyl grouping,

$R_2$ represents:

. a linear or branched lower alkyl grouping optionally substituted by one or more halogen atoms or by one or more alkoxy, hydroxy, phenyl, carboxylic acid groupings,

. a phenyl grouping optionally substituted by one or more:
  - halogen atoms, or
  - lower alkyl groupings optionally substituted by one or more halogen atoms, or
  - hydroxy or lower alkoxy groupings,

. a linear or branched lower alkenyl grouping optionally substituted by a carboxylic acid, alkoxy, hydroxy or aryl grouping,

. a 2-thienyl grouping optionally substituted by a linear or branched lower alkyl grouping,

. a 2-furyl grouping optionally substituted by a linear or branched lower alkyl grouping,

. a 2-pyrrolyl grouping optionally substituted by a linear or branched lower alkyl grouping, or

. a pyridyl grouping optionally substituted by a linear or branched lower alkyl grouping,

X represents a hydrogen atom,

Y represents a hydroxy grouping,
or X and Y together represent an oxygen atom,
the term "lower" indicating that the groupings so qualified contain from 1 to 6 carbon atoms,
the term "lower" qualifying alkenyl indicating a grouping having up to 6 carbon atoms,
their enantiomers, diastereoisomers and epimers and also, when $R_1$ represents a hydrogen atom and/or $R_2$ contains a carboxylic acid grouping, their addition salts with a pharmaceutically acceptable base, and also, when $R_2$ contains an amino grouping, their addition salts with a pharmaceutically acceptable acid, characterised in that there is used as starting material a compound of formula (II):

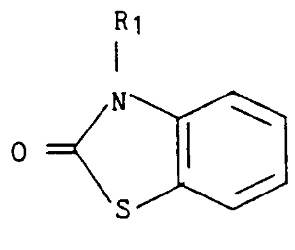

(II)

in which $R_1$ is as defined in formula (I),
which is subjected, depending on the nature of $R_2$,

* either to the action of an acid chloride of formula (III):

$$R_2 - CO - Cl \qquad (III)$$

in which $R_2$ is as defined in formula (I),
or the corresponding acid anhydride or lactone,
preferably in the presence of aluminium chloride in dimethylformamide,
to obtain a compound of formula (I/a):

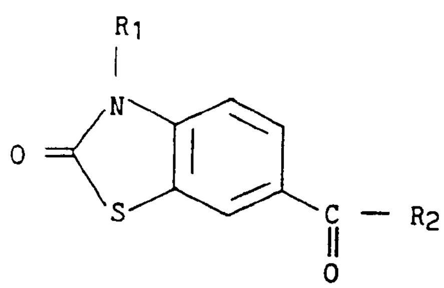

(I/a)

a particular case of the compounds of formula (I),
in which:
$R_1$ and $R_2$ are as defined in formula (I), and
X and Y together represent an oxygen atom,

* or to the action of an acid of formula (V):

$$R_2 - CO_2H \qquad (V)$$

in which $R_2$ is as defined in formula (I),
or the corresponding acid chloride or acid anhydride or lactone in the presence of polyphosphoric acid
to obtain a compound of formula (I/a):

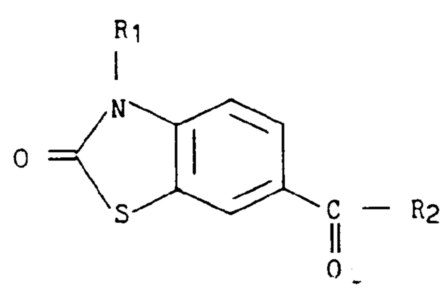

(I/a)

a particular case of the compounds of formula (I), in which: $R_1$ and $R_2$ are as defined in formula (I), and X and Y together represent an oxygen atom, which compound of formula (I/a) is optionally purified by a conventional purification technique and, if desired, in the cases where $R_1$ represents a hydrogen atom, or in the cases where $R_2$ contains a carboxylic acid grouping or an amino grouping, may be converted into a salt by means of a pharmaceutically acceptable base or acid, respectively, and which, when $R_2$ represents an aryl grouping substituted by one or more lower alkoxy groupings, may be subjected, if desired, to the action of a strong acid to yield a compound of formula (I/z):

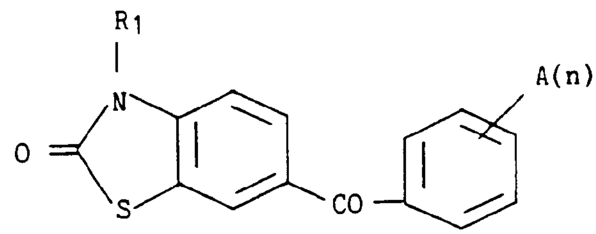

(I/z)

in which $R_1$ is as defined in formula (I), A represents a hydroxy grouping and n is an integer from 1 to 5, the position of the hydroxy grouping(s) being identical with that of the lower alkoxy grouping(s) of the product of formula (I) that was subjected to the action of the acid, which product of formula (I/z) is optionally purified by a conventional technique and, if desired, in the cases where $R_1$ represents a hydrogen atom, may be converted into a salt by means of a pharmaceutically acceptable base, which compound of formula (I/a) or of formula (I/z) may, if desired, be subjected to the action of a hydrogenation agent selected from mixed alkali metal hydrides, such as, for example, sodium borohydride, preferably in the presence of a lower aliphatic alcohol, to yield a compound of formula (I/b):

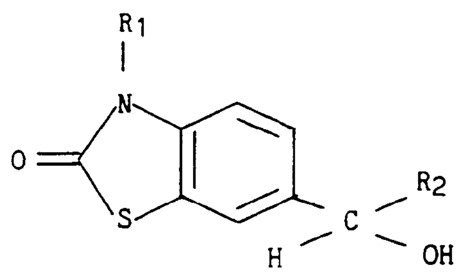

(I/b)

a particular case of the compounds of formula (I) in which: $R_1$ and $R_2$ are as defined in formula (I), Xrepresents a hydrogen atom, and Y represents a hydroxy grouping, the isomers of which are, if desired, separated by a conventional separation technique and which is optionally purified by a conventional purification technique and which, in the case where $R_1$ represents a hydrogen atom or in the case where $R_2$ contains a carboxylic acid grouping, may be converted into a salt by means of a pharmaceutically acceptable base or which, in the cases where $R_2$ contains an amino grouping, may be converted into a salt by means of a pharmaceutically acceptable acid.

2. Process for the preparation according to claim 1 of compounds of formula (I/z):

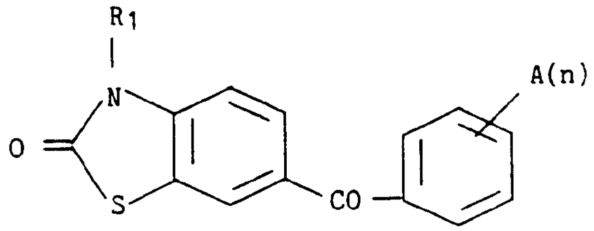

(I/z)

a particular case of the compounds of formula (I) in which $R_1$ is as defined in formula (I), A represents a hydroxy grouping and n is an integer from 1 to 5, characterised in that a compound of formula (I) according to claim 1 in which the $R_2$ grouping represents a phenyl radical substituted by from 1 to 5 lower alkoxy groupings is subjected to the action of a strong acid,
to yield a compound of formula (I/z), the position of the hydroxy groupings of the compound so obtained being identical with that of the lower alkoxy groupings of the product of formula (I) that was subjected to the strong acid,
which product of formula (I/z) is optionally purified by a conventional technique and, in the case where $R_1$ represents a hydrogen atom, may, if desired, be converted into a salt by means of a pharmaceutically acceptable base.

3. Process for the preparation according to claim 1 of compounds of formula (I) in which X and Y together represent an oxygen atom, their enantiomers, diastereoisomers and epimers and also, when $R_1$ represents a hydrogen atom and/or $R_2$ contains a carboxylic acid grouping, their addition salts with a pharmaceutically acceptable base and also, when $R_2$ contains an amino grouping, their addition salts with a pharmaceutically acceptable acid.

4. Process for the preparation according to claim 1 of compounds of formula (I) in which $R_1$ represents a hydrogen atom, their enantiomers, diastereoisomers and epimers and also their addition salts with a pharmaceutically acceptable base and, when $R_2$ contains an amino grouping, their addition salts with a pharmaceutically acceptable acid.

5. Process for the preparation according to claim 1 of compounds of formula (I) in which $R_1$ represents a methyl grouping, their enantiomers, diastereoisomers and epimers and also, when $R_2$ contains a carboxylic acid grouping, their addition salts with a pharmaceutically acceptable base and, when $R_2$ contains an amino grouping, their addition salts with a pharmaceutically acceptable acid.

6. Process for the preparation according to claim 1 of compounds of formula (I) in which $R_2$ represents a phenyl grouping, their enantiomers, diastereoisomers and epimers and also, when $R_1$ represents a hydrogen atom, their addition salts with a pharmaceutically acceptable base.

7. Process for the preparation according to claim 1 of compounds of formula (I) in which $R_2$ represents an alkyl grouping, their enantiomers, diastereoisomers and epimers, hydrogen atom, and also, when $R_1$ represents a hydrogen atom, their addition salts with a pharmaceutically acceptable base.

8. Process for the preparation according to claim 1 of the compound of formula (I) which is 6-benzoylbenzothiazolinone, and also its addition salts with a pharmaceutically acceptable base.

9. Process for the preparation according to claim 1 of compounds of formula (I) in which $R_2$ represents a phenyl grouping, and also, when $R_1$ represents a hydrogen atom, their addition salts with a pharmaceutically acceptable base.

10. Process for the preparation according to claim 1 of compounds of formula (I) in which $R_2$ represents a phenyl grouping substituted by one or more halogen atoms, and also, when $R_1$ represents a hydrogen atom, their addition salts with a pharmaceutically acceptable base.

11. Process for the preparation according to claim 1 of compounds of formula (I) in which $R_2$ represents a thienyl grouping, and also, when $R_1$ represents a hydrogen atom, their addition salts with a pharmaceutically acceptable base.

12. Process for the preparation according to claim 1 of compounds of formula (I) in which $R_2$ represents a lower alkyl or phenyl grouping substituted by a hydroxy grouping, their enantiomers, diastereoisomers and epimers and also, when $R_1$ represents a hydrogen atom, their addition salts with a pharmaceutically acceptable base.

13. Process for the preparation according to claim 1 of compounds of formula (I) in which $R_2$ represents a lower alkyl grouping substituted by a halogen atom, their enantiomers, diastereoisomers and epimers and also, when $R_1$ represents a hydrogen atom, their addition salts with a pharmaceutically acceptable base.

14. Process for the preparation according to claim 1 of compounds of formula (I) in which $R_2$ represents a lower alkyl grouping substituted by a carboxylic acid grouping, their enantiomers, diastereoisomers and epimers and also their addition salts with a pharmaceutically acceptable base.

15. Process for the preparation according to claim 1 of compounds of formula (I) in which $R_2$ represents a lower alkenyl grouping substituted by a carboxylic acid grouping, their enantiomers, diastereoisomers and epimers and also their addition salts with a pharmaceutically acceptable base.

16. Process for the preparation according to claim 1 of compounds of formula (I) in which $R_2$ represents a pyridyl grouping, their enantiomers, diastereoisomers and epimers and also their addition salts with a pharmaceutically acceptable acid and, when $R_1$ represents a hydrogen atom, their addition salts with a pharmaceutically acceptable base.

17. Process for the preparation according to claim 1 of compounds of formula (I) in which X represents a hydrogen atom, Y represents a hydroxy grouping, their enantiomers, diastereoisomers and epimers and also, when $R_1$ represents a hydrogen atom, their addition salts with a pharmaceutically acceptable base and, when $R_2$ contains an amino grouping, their addition salts with a pharmaceutically acceptable acid.

18. Process for the preparation according to claim 1 of compounds of formula (I) in which $R_2$ represents a lower alkylphenyl grouping, their enantiomers, diastereoisomers and epimers and also, when $R_1$ represents a hydrogen atom, their addition salts with a pharmaceutically acceptable base.

19. Process for the preparation according to either claim 1 or claim 2 of compounds of formula (I) in which $R_2$ represents a phenyl grouping substituted by one or more groupings selected from lower alkyl, lower alkoxy, hydroxy and trifluoromethyl, their isomers, diastereoisomers and epimers and also, when $R_1$ represents a hydrogen atom, their addition salts with a pharmaceutically acceptable base.

20. Process for the preparation of pharmaceutical compositions containing as active ingredient at least one compound obtained according to any one of claims 1 to 19 in combination with one or more pharmaceutically acceptable, inert, non-toxic excipients or carriers.

## Patentansprüche

### Patentansprüche für folgende Vertragsstaaten : AT, BE, CH, DE, DK, FR, GB, IT, LI, LU, NL, SE

1. Verbindungen der allgemeinen Formel (I):

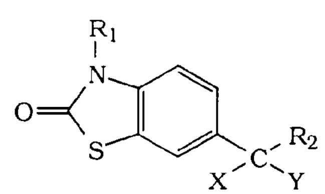

(I)

in der

$R_1$ ein Wasserstoffatom oder eine Niedrigalkylgruppe,

$R_2$

. eine geradkettige oder verzweigte Niedrigalkylgruppe, die gegebenenfalls durch eines oder mehrere Halogenatome oder durch eine oder mehrere Alkoxygruppen, Hydroxylgruppen, Phenylgruppen

oder Carbonsäuregruppen substituiert ist,

. eine Phenylgruppe, die gegebenenfalls durch eine oder mehrere
  - Halogenatome oder
  - Niedrigalkylgruppen, die gegebenenfalls durch eines oder mehrere Halogenatome substituiert sind, oder
  - Hydroxylgruppen oder Niedrigalkoxygruppen substituiert ist,

. eine geradkettige oder verzweigte Niedrigalkenylgruppe, die gegebenenfalls durch eine Carbonsäuregruppe, Alkoxygruppe, Hydroxylgruppe oder Arylgruppe substituiert ist,

. eine Thien-2-yl-gruppe, die gegebenenfalls durch eine geradkettige oder verzweigte Niedrigalkylgruppe substituiert ist,

. eine Fur-2-yl-gruppe, die gegebenenfalls durch eine geradkettige oder verzweigte Niedrigalkylgruppe substituiert ist,

. eine Pyrrol-2-yl-gruppe, die gegebenenfalls durch eine geradkettige **oder** verzweigte Niedrigalkylgruppe substituiert ist,

. eine Pyridylgruppe, die gegebenenfalls durch eine geradkettige oder verzweigte Niedrigalkylgruppe substituiert ist,

X eine Wasserstoffatom und
Y ein Hydroxylgruppe oder
X und Y gemeinsam ein Sauerstoffatom bedeuten,
wobei der Begriff "Niedrig" bedeutet, daß die in dieser Weise bezeichneten Gruppen 1 bis 6 Kohlenstoffatome aufweisen und der Begriff "Niedrigalkenyl" bedeutet, daß die Gruppe bis zu 6 Kohlenstoffatome besitzt,
deren Enantiomere. Diastereoisomere und Epimere sowie, wenn $R_1$ ein Wasserstoffatom bedeutet und/oder $R_2$ eine Carbonsäuregruppe umfaßt, deren Additionssalze mit einer pharmazeutisch annehmbaren Base sowie, wenn $R_2$ eine stickstoffhaltige Gruppe umfaßt, deren Additionssalze mit einer pharmazeutisch annehmbaren Säure.

**2.** Verbindungen nach Anspruch 1, worin X und Y gemeinsam ein Sauerstoffatom bedeuten. deren Enantiomere. Diastereoisomere und Epimere sowie, wenn $R_1$ ein Wasserstoffatom bedeutet und/oder $R_2$ eine Carbonsäuregruppe umfaßt, deren Additionssalze mit einer pharmazeutisch annehmbaren Base sowie, wenn $R_2$ eine stickstoffhaltige Gruppe umfaßt, deren Additionssalze mit einer pharmazeutisch annehmbaren Säure.

**3.** Verbindungen nach Anspruch 1, worin $R_1$ ein Wasserstoffatom bedeutet, deren Enantiomere. Diastereoisomere und Epimere sowie deren Additionssalze mit einer pharmazeutisch annehmbaren Base und, wenn $R_2$ eine stickstoffhaltige Gruppe umfaßt, deren Additionssalze mit einer pharmazeutisch annehmbaren Säure.

**4.** Verbindungen nach Anspruch 1, worin $R_1$ eine Methylgruppe bedeutet, deren Enantiomere, Diastereoisomere und Epimere sowie, wenn $R_2$ eine Carbonsäuregruppe umfaßt, deren Additionssalze mit einer pharmazeutisch annehmbaren Base und, wenn $R_2$ eine stickstoffhaltige Gruppe umfaßt, deren Additionssalze mit einer pharmazeutisch annehmbaren Säure.

**5.** Verbindungen nach Anspruch 1, worin $R_2$ eine Phenylgruppe bedeutet, deren Enantiomere, Diastereoisomere, Epimere sowie dann, wenn $R_1$ ein Wasserstoffatom bedeutet, deren Additionssalze mit einer pharmazeutisch annehmbaren Base.

**6.** Verbindungen nach Anspruch 1, worin $R_2$ eine Alkylgruppe bedeutet, deren Enantiomere. Diastereoisomere und Epimere sowie, wenn $R_1$ ein Wasserstoffatom bedeutet, deren Additionssalze mit einer pharmazeutisch annehmbaren Base.

**7.** Verbindungen nach Anspruch 1, nämlich 6-Benzoyl-benzothiazolinon, sowie dessen Additionssalze mit einer pharmazeutisch annehmbaren Base.

**8.** Verbindungen nach Anspruch 1, worin $R_2$ eine Phenylgruppe bedeutet, sowie, wenn $R_1$ ein Wasserstoffatom bedeutet, deren Additionssalze mit einer pharmazeutisch annehmbaren Base.

**9.** Verbindungen nach Anspruch 1, worin $R_2$ eine Phenylgruppe, die durch eines oder mehrere Halogenatome substituiert ist, bedeutet und, wenn $R_1$ ein Wasserstoffatom darstellt, deren Additionssalze mit einer

pharmazeutisch annehmbaren Base.

**10.** Verbindungen nach Anspruch 1, worin $R_2$ eine Thienylgruppe bedeutet, sowie, wenn $R_1$ ein Wasserstoffatom bedeutet, deren Additionssalze mit einerpharmazeutisch annehmbaren Base.

**11.** Verbindungen nach Anspruch 1, worin $R_2$ eine Niedrigalkylgruppe oder eine Phenylgruppe, die durch eine Hydroxylgruppe substituiert ist, bedeutet, deren Enantiomere, Diastereoisomere und Epimere sowie, wenn $R_1$ ein Wasserstoffatom darstellt, deren Additionssalze mit einer pharmazeutisch annehmbaren Base.

**12.** Verbindungen nach Anspruch 1, worin $R_2$ eine durch ein Halogenatom substituierte Niedrigalkylgruppe bedeutet, deren Enantiomere, Diastereoisomere und Epimere sowie, wenn $R_1$ ein Wasserstoffatom darstellt, deren Additionssalze mit einer pharmazeutisch annehmbaren Base.

**13.** Verbindungen nach Anspruch 1, worin $R_2$ eine durch eine Carbonsäuregruppe substituierte Niedrigalkylgruppe bedeutet, deren Enantiomere, Diastereoisomere und Epimere sowie deren Additionssalze mit einer pharmazeutisch annehmbaren Base.

**14.** Verbindungen nach Anspruch 1, worin $R_2$ eine durch eine Carbonsäuregruppe substituierte Niedrigalkenylgruppe bedeutet, deren Enantiomere, Diastereoisomere und Epimere sowie deren Additionssalze mit einer pharmazeutisch annehmbaren Base.

**15.** Verbindungen nach Anspruch 1, worin $R_2$ eine Pyridylgruppe bedeutet, deren Enantiomere, Diastereoisomere und Epimere sowie deren Additionssalze mit einer pharmazeutisch annehmbaren Säure und, wenn $R_1$ ein Wasserstoffatom darstellt. deren Additionssalze mit einer pharmazeutisch annehmbaren Base.

**16.** Verbindungen nach Anspruch 1, worin X ein Wasserstoffatom und Y eine Hydroxylgruppe bedeuten, deren Enantiomere, Diastereoisomere und Epimere sowie, wenn $R_1$ ein Wasserstoffatom darstellt, deren Additionssalze mit einer pharmazeutisch annehmbaren Base und, wenn $R_2$ eine stickstoffhaltige Gruppe umfaßt, deren Additionssalze mit einer pharmazeutisch annehmbaren Säure.

**17.** Verbindungen nach Anspruch 1, worin $R_2$ eine Niedrigalkylphenylgruppe bedeutet, deren Enantiomere, Diastereoisomere und Epimere sowie, wenn $R_1$ ein Wasserstoffatom darstellt. deren Additionssalze mit einer pharmazeutisch annehmbaren Base.

**18.** Verbindungen nach Anspruch 1, worin $R_2$ eine Phenylgruppe, die durch eine oder mehrere Gruppen, ausgewählt aus Niedrigalkylgruppen, Niedrigalkoxygruppen. Hydroxylgruppen und Trifluromethylgruppen, substituiert ist, bedeutet, deren Isomere, Diastereoisomere und Epimere sowie, wenn $R_1$ ein Wasserstoffatom darstellt, deren Additionssalze mit einer pharmazeutisch annehmbaren Base.

**19.** Verfahren zur Herstellung der Verbindungen der allgemeinen Formel (I), **dadurch gekennzeichnet**, daß man als Ausgangsmaterial ein Derivat der Formel (II) verwendet:

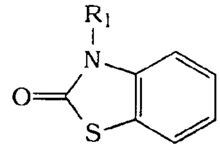

(II)

in der $R_1$ die bezüglich der Formel (I) angegebenen Bedeutungen besitzt, die man in Abhängigkeit von der Art der Gruppe $R_2$,

* entweder der Einwirkung eines Säurechlorids der Formel (III):

$$R_2 - CO - Cl \qquad (III)$$

worin $R_2$ die bezüglich der Formel (I) angegebenen Bedeutungen besitzt, oder des entsprechenden Säureanhydrids oder Lactons

vorzugsweise in Gegenwart von Aluminiumchlorid in Dimethylformamid unterwirft,

so daß man ein Derivat der Formel (I/a) erhält:

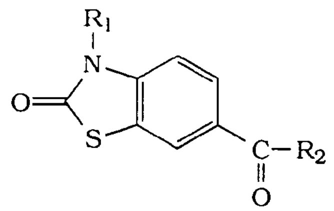

(I/a)

einem Sonderfall der Derivate der Formel (I),
in der:

$R_1$ und $R_2$ die bezüglich der Formel (I) angegebenen Bedeutungen besitzen und
X und Y gemeinsam ein Sauerstoffatom darstellen,

* oder der Einwirkung einer Säure der Formel (V):

$$R_2 - CO_2H \qquad (V)$$

in der $R_2$ die bezüglich der Formel (I) angegebenen Bedeutungen besitzt. oder des entsprechenden Säurechlorids oder Säureanhydrids oder Lactons in Gegenwart von Polyphosphorsäure unterwirft, so daß man man ein Derivat der Formel (I/a) erhält:

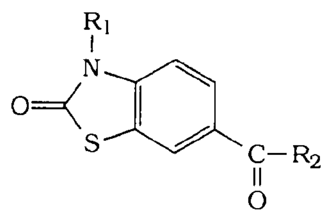

(I/a)

einem Sonderfall der Derivate der Formel (I),
in der:

$R_1$ und $R_2$ die bezüglich der Formel (I) angegebenen Bedeutungen besitzen und
X und Y gemeinsam ein Sauerstoffatom darstellen,

welches Derivat der Formel (I/a) man gegebenenfalls mit Hilfe einer klassischen Reinigungsmethode reinigt und welches man gewünschtenfalls dann, wenn $R_1$ ein Wasserstoffatom darstellt oder dann, wenn $R_2$ eine Carbonsäuregruppe oder eine Aminogruppe umfaßt, mit einer pharmazeutisch annehmbaren Base oder Säure in ein Salz überführen kann,
und welches, wenn $R_2$ eine durch eine oder mehrere Niedrigalkoxygruppen substituierte Arylgruppe darstellt, gewünschtenfalls der Einwirkung einer starken Säure unterworfen werden kann, so daß man ein Derivat der Formel (I/z) erhält:

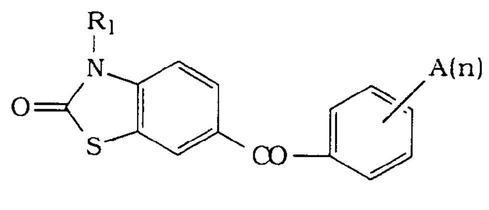

(I/z)

worin $R_1$ die bezüglich der Formel (I) angegebenen Bedeutungen besitzt, A eine Hydroxylgruppe darstellt und n eine ganze Zahl mit einem Wert zwischen 1 und 5 bedeutet,
wobei die Stellung der Hydroxylgruppe(n) identisch ist mit jener der Niedrigalkoxygruppe(n) des Produkts der Formel (1), welches der Einwirkung der Säure unterworfen worden ist,
welches Produkt der Formel (I/z) man gegebenenfalls mit Hilfe einer klassischen Methode reinigt und welches man, wenn $R_1$ ein Wasserstoffatm darstellt, gewünschtenfalls mit einer pharmazeutisch annehmbaren Base in ein Salz überführen kann,
welches Derivat der Formel (I/a) oder der Formel (I/z) gewünschtenfalls der Einwirkung eines aus einem gemischten Alkalimetallhydrid, wie beispielsweise Natriumborhydrid, ausgewählten Hydrierungsmittels vorzugsweise in Gegenwart eines niedrigmolekularen aliphatischen Alkohols unterworfen werden kann, so daß man ein Derivat der Formel (I/b) erhält:

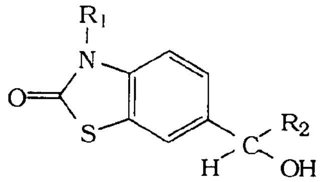

(I/b)

einem Sonderfall der Derivate der Formel (I), in der

$R_1$ und $R_2$ die bezüglich der Formel (I) angegebenen Bedeutungen besitzen,

X ein Wasserstoffatom und

Y eine Hydroxylgruppe bedeuten,

welches man gewünschtenfalls mit Hilfe einer klassischen Trennmethode in die Isomeren auftrennen und gegebenenfalls mit Hilfe einer klassischen Reinigungsmethode reinigen kann und - wenn $R_1$ ein Wasserstoffatom bedeutet oder wenn $R_2$ eine Carbonsäuregruppe umfaßt, mit einer pharmazeutisch annehmbaren Base in ein Salz überführen oder, wenn $R_2$ eine stickstoffhaltige Gruppe umfaßt, mit einer pharmazeutisch annehmbaren Säure in ein Salz überführen kann.

**20.** Verfahren nach Anspruch 19 zur Herstellung von Verbindungen der Formel (I/z):

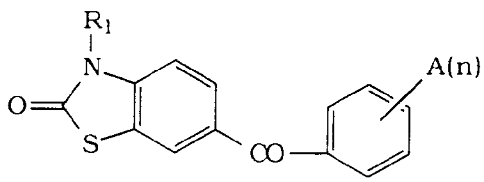

(I/z)

einem Sonderfall der Derivate der Formel (I), in der $R_1$ die bezüglich der Formel (I) angegebenen Bedeutungen besitzt, A eine Hydroxylgruppe und n eine ganze Zahl mit einem Wert zwischen 1 und 5 bedeuten, **dadurch gekennzeichnet**, daß man ein Derivat der Formel (I) nach Anspruch 1, worin die Gruppe $R_2$ eine Phenylgruppe, die durch 1 bis 5 Niedrigalkoxygruppen substituiert ist, bedeutet, der Einwirkung einer starken Säure unterwirft,

so daß man ein Derivat der Formel (I/z) erhält, in dem die Stellung der Hydroxylgruppen des in dieser Weise erhaltenen Derivats identisch ist mit jener der Niedrigalkoxygruppen des der Einwirkung der starken Säure unterworfenen Produkts der Formel (I),

welches Produkt der Formel (I/z) man gegebenenfalls mit Hilfe einer klassischen Methode reinigt und welches man dann, wenn $R_1$ ein Wasserstoffatom darstellt, gewünschtenfalls mit Hilfe einer pharmazeutisch annehmbaren Base in ein Salz überführen kann.

**21.** Pharmazeutische Zubereitung enthaltend als Wirkstoff mindestens eine Verbindung nach einem der Ansprüche 1 bis 18 in Kombination mit einem oder mehreren inerten, nichttoxischen, pharmazeutisch annehmbaren Bindemitteln oder Trägermaterialien,

**22.** Pharmazeutische Zubereitung nach Anspruch 21 enthaltend mindestens einen Wirkstoff nach einem der Ansprüche 1 bis 18, welches als Analgetikum oder als normolipämisches Mittel und zur Behandlung von ischämischen arteriellen Erkrankungen eingesetzt werden kann.

**Patentansprüche für folgende Vertragsstaaten : ES, GR**

**1.** Verfahren zur Herstellung von Verbindungen der allgemeinen Formel (I):

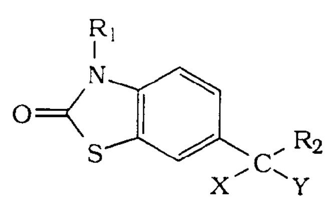

(I)

in der
$R_1$ ein Wasserstoffatom oder eine Niedrigalkylgruppe,
$R_2$

- eine geradkettige oder verzweigte Niedrigalkylgruppe, die gegebenenfalls durch eines oder mehrere Halogenatome oder durch eine oder mehrere Alkoxygruppen, Hydroxylgruppen, Phenylgruppen oder Carbonsäuregruppen substituiert ist,
- eine Phenylgruppe, die gegebenenfalls durch eine oder mehrere
  - Halogenatome oder
  - Niedrigalkylgruppen, die gegebenenfalls durch eines oder mehrere Halogenatome substituiert sind, oder
  - Hydroxylgruppen oder Niedrigalkoxygruppen substituiert ist.
- eine geradkettige oder verzweigte Niedrigalkenylgruppe, die gegebenenfalls durch eine Carbonsäuregruppe, Alkoxygruppe, Hydroxylgruppe oder Arylgruppe substituiert ist.
- eine Thien-2-yl-gruppe, die gegebenenfalls durch eine geradkettige oder verzweigte Niedrigalkylgruppe substituiert ist,
- eine Fur-2-yl-gruppe, die gegebenenfalls durch eine geradkettige oder verzweigte Niedrigalkylgruppe substituiert ist,
- eine Pyrrol-2-yl-gruppe, die gegebenenfalls durch eine geradkettige oder verzweigte Niedrigalkylgruppe substituiert ist.
- eine Pyridylgruppe, die gegebenenfalls durch eine geradkettige oder verzweigte Niedrigalkylgruppe substituiert ist,

X eine Wasserstoffatom und
Y eine Hydroxylgruppe oder
X und Y gemeinsam ein Sauerstoffatom bedeuten,
wobei der Begriff "Niedrig" bedeutet, daß die in dieser Weise bezeichneten Gruppen 1 bis 6 Kohlenstoffatome aufweisen und der Begriff "Niedrigalkenyl" bedeutet, daß die Gruppe bis zu 6 Kohlenstoffatome besitzt,
von deren Enantiomeren, Diastereoisomeren und Epimeren sowie, wenn $R_1$ ein Wasserstoffatom bedeutet und/oder $R_2$ eine Carbonsäuregruppe umfaßt, deren Additionssalzen mit einer pharmazeutisch annehmbaren Base sowie, wenn $R_2$ eine stickstoffhaltige Gruppe umfaßt, von deren Additionssalzen mit einer pharmazeutisch annehmbaren Säure,
**dadurch gekennzeichnet**, daß man als Ausgangsmaterial ein Derivat der Formel (II) verwendet:

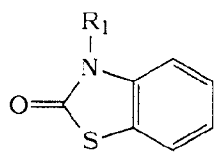

(II)

in der $R_1$ die bezüglich der Formel (I) angegebenen Bedeutungen besitzt, die man in Abhängigkeit von der Art der Gruppe $R_2$,

* entweder der Einwirkung eines Säurechlorids der Formel (III):

$$R_2 - CO - Cl \quad (III)$$

worin $R_2$ die bezüglich der Formel (I) angegebenen Bedeutungen besitzt, oder des entsprechenden Säureanhydrids oder Lactons
vorzugsweise in Gegenwart von Aluminiumchlorid in Dimethylformamid unterwirft,
so daß man ein Derivat der Formel (I/a) erhält:

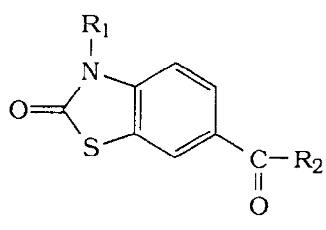

(I/a)

einem Sonderfall der Derivate der Formel (I),
in der:

$R_1$ und $R_2$ die bezüglich der Formel (I) angegebenen Bedeutungen besitzen und
X und Y gemeinsam ein Sauerstoffatom darstellen,

* oder der Einwirkung einer Säure der Formel (V):

$$R_2 - CO_2H \qquad (V)$$

in der $R_2$ die bezüglich der Formel (I) angegebenen Bedeutungen besitzt, oder des entsprechenden Säurechlorids oder Säureanhydrids oder Lactons in Gegenwart von Polyphosphorsäure unterwirft, so daß man ein Derivat der Formel (I/a) erhält:

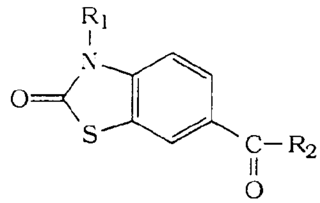

(I/a)

einem Sonderfall der Derivate der Formel (I),
in der:

$R_1$ und $R_2$ die bezüglich der Formel (I) angegebenen Bedeutungen besitzen und
X und Y gemeinsam ein Sauerstoffatom darstellen, welches Derivat der Formel (I/a) man gegebenenfalls mit Hilfe einer klassischen Reinigungsmethode reinigt und welches man gewünschtenfalls dann, wenn $R_1$ ein Wasserstoffatom darstellt oder dann, wenn $R_2$ eine Carbonsäuregruppe oder eine Aminogruppe umfaßt, mit einer pharmazeutisch annehmbaren Base oder Säure in ein Salz überführen kann,

und welches, wenn $R_2$ eine durch eine oder mehrere Niedrigalkoxygruppen substituierte Arylgruppe darstellt, gewünschtenfalls der Einwirkung einer starken Säure unterworfen werden kann, so daß man ein Derivat der Formel (I/z) erhält:

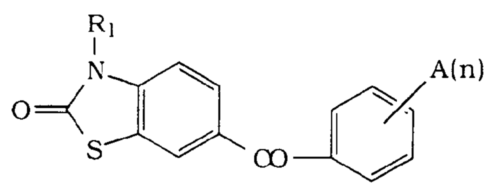

(I/z)

worin $R_1$ die bezüglich der Formel (I) angegebenen Bedeutungen besitzt, A eine Hydroxylgruppe darstellt und n eine ganze Zahl mit einem Wert zwischen 1 und 5 bedeutet,
wobei die Stellung der Hydroxylgruppe(n) identisch ist mit jener der Niedrigalkoxygruppe(n) des Produkts der Formel (I), welches der Einwirkung der Säure unterworfen worden ist,
welches Produkt der Formel (I/z) man gegebenenfalls mit Hilfe einer klassischen Methode reinigt und welches man, wenn $R_1$ ein Wasserstoffatm darstellt, gewünschtenfalls mit einer pharmazeutisch annehmbaren Base in ein Salz überführen kann,
welches Derivat der Formel (I/a) oder der Formel (I/z) gewünschtenfalls der Einwirkung eines aus einem gemischten Alkalimetallhydrid, wie beispielsweise Natriumborhydrid, ausgewählten Hydrierungsmittels vorzugsweise in Gegenwart eines niedrigmolekularen aliphatischen Alkohols unterworfen werden kann, so daß man ein Derivat der Formel (I/b) erhält:

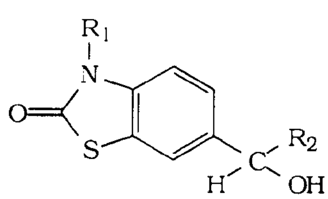

(I/b)

einem Sonderfall der Derivate der Formel (I), in der

$R_1$ und $R_2$ die bezüglich der Formel (I) angegebenen Bedeutungen besitzen,
X ein Wasserstoffatom und

Y eine Hydroxylgruppe bedeuten,
welches man gewünschtenfalls mit Hilfe einer klassischen Trennmethode in die Isomeren auftrennen und gegebenenfalls mit Hilfe einer klassischen Reinigungsmethode reinigen kann und - wenn $R_1$ ein Wasserstoffatom bedeutet oder, wenn $R_2$ eine Carbonsäuregruppe umfaßt, mit einer pharmazeutisch annehmbaren Base in ein Salz überführen oder, wenn $R_2$ eine stickstoffhaltige Gruppe umfaßt, mit einer pharmazeutisch annehmbaren Säure in ein Salz überführen kann.

2. Verfahren nach Anspruch 1 zur Herstellung von Verbindungen der Formel [I/z]:

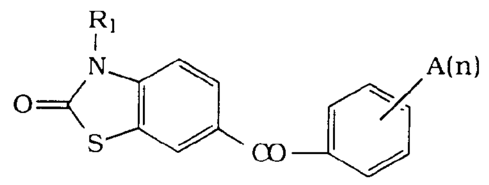

(I/z)

einem Sonderfall der Derivate der Formel (I), in der $R_1$ die bezüglich der Formel (I) angegebenen Bedeutungen besitzt, A eine Hydroxylgruppe und n eine ganze Zahl mit einem Wert zwischen 1 und 5 bedeuten, **dadurch gekennzeichnet**, daß man ein Derivat der Formel (I) nach Anspruch 1, worin die Gruppe $R_2$ eine Phenylgruppe, die durch 1 bis 5 Niedrigalkoxygruppen substituiert ist, bedeutet, der Einwirkung einer starken Säure unterwirft,
so daß man ein Derivat der Formel (I/z) erhält, in dem die Stellung der Hydroxylgruppen des in dieser Weise erhaltenen Derivats identisch ist mit jener der Niedrigalkoxygruppen des der Einwirkung der starken Säure unterworfenen Produkts der Formel (I),
welches Produkt der Formel (I/z) man gegebenenfalls mit Hilfe einer klassischen Methode reinigt und welches man dann, wenn $R_1$ ein Wasserstoffatom darstellt, gewünschtenfalls mit Hilfe einer pharmazeutisch annehmbaren Base in ein Salz überführen kann.

3. Verfahren nach Anspruch 1 zur Herstellung von Verbindungen der Formel (I), in der X und Y gemeinsam ein Sauerstoffatom bedeuten, von deren Enantiomeren, Diastereoisomeren und Epimeren sowie, wenn $R_1$ ein Wasserstoffatom darstellt und/oder $R_2$ eine Carbonsäuregruppe umfaßt, von deren Additionssalzen mit einer pharmazeutisch annehmbaren Base, sowie, wenn $R_2$ eine stickstoffhaltige Gruppe umfaßt, deren Additionssalzen mit einer pharmazeutisch annehmbaren Säure.

4. Verfahren nach Anspruch 1 zur Herstellung von Verbindungen der Formel (I), worin $R_1$ ein Wasserstoffatom darstellt, von deren Enantiomeren, Diastereoisomeren und Epimeren sowie von deren Additionssalzen mit einer pharmazeutisch annehmbaren Base und, wenn $R_2$ eine stickstoffhaltige Gruppe umfaßt, von deren Additionssalzen mit eine pharmazeutisch annehmbaren Säure.

5. Verfahren nach Anspruch 1 zur Herstellung von Verbindungen der Formel (I), worin $R_1$ eine Methylgruppe bedeutet, von deren Enantiomeren, Diastereoisomeren und Epimeren sowie, wenn $R_2$ eine Carbonsäuregruppe umfaßt, deren Additionssalzen mit einer pharmazeutisch annehmbaren Base und, wenn $R_2$ eine stickstoffhaltige Gruppe umfaßt, von deren Additionssalzen mit einer pharmazeutisch annehmbaren Säure.

6. Verfahren nach Anspruch 1 zur Herstellung von Verbindungen der Formel (I), worin $R_2$ eine Phenylgruppe bedeutet, von deren Enantiomeren, Diastereoisomeren und Epimeren sowie, wenn $R_1$ ein Wasserstoffatom darstellt, von deren Additionssalzen mit einer pharmazeutisch annehmbaren Base.

7. Verfahren nach Anspruch 1 zur Herstellung von Verbindungen der Formel (I), worin $R_2$ eine Alkylgruppe bedeutet, von deren Enantiomeren, Diastereoisomeren und Epimeren sowie, wenn $R_1$ ein Wasserstoffatom darstellt, von deren Additionssalzen mit einer pharmazeutisch annehmbaren Base.

8. Verfahren nach Anspruch 1 zur Herstellung von Verbindungen der Formel (I), nämlich 6-Benzoylbenzothiozolinon, sowie von deren Additionssalzen mit einer pharmazeutisch annehmbaren Base.

9. Verfahren nach Anspruch 1 zur Herstellung von Verbindungen der Formel (I), in der $R_2$ eine Phenylgruppe darstellt, sowie, wenn $R_1$ ein Wasserstoffatom darstellt, von deren Additionssalzen mit einer pharmazeutisch annehmbaren Base.

10. Verfahren nach Anspruch 1 zur Herstellung von Verbindungen der Formel (I), in der $R_2$ eine Phenylgruppe darstellt, die durch eines oder mehrere Halogenatome substituiert ist, sowie, wenn $R_1$ ein Wasserstoffatom darstellt, von deren Additionssalzen mit einer pharmazeutisch annehmbaren Base.

11. Verfahren nach Anspruch 1 zur Herstellung von Verbindungen der Formel (I), in der $R_2$ eine Thienylgruppe darstellt, sowie, wenn $R_1$ ein Wasserstoffatom darstellt, von deren Additionssalzen mit einer pharmazeutisch annehmbaren Base.

12. Verfahren nach Anspruch 1 zur Herstellung von Verbindungen der Formel (I), in der $R_2$ eine Niedrigalkylgruppe oder eine Phenylgruppe, die durch eine Hydroxylgruppe substituiert ist, bedeutet, sowie von deren Enantiomeren, Diastereoisomeren und Epimeren sowie, wenn $R_1$ ein Wasserstoffatom bedeutet, von deren Additionssalzen mit einer pharmazeutisch annehmbaren Base.

13. Verfahren nach Anspruch 1 zur Herstellung von Verbindungen der Formel (I), worin $R_2$ eine durch ein Halogenatom substituierte Niedrigalkylgruppe darstellt, von deren Enantiomeren, Diastereoisomeren und Epimeren sowie, wenn $R_1$ ein Wasserstoffatom bedeutet, von deren Additionssalzen mit einer pharmazeutisch annehmbaren Base.

14. Verfahren nach Anspruch 1 zur Herstellung von Verbindungen der Formel (I), in der $R_2$ eine durch eine Carbonsäuregruppe substituierte Niedrigalkylgruppe darstellt, von deren Enantiomeren, Diastereoisomeren und Epimeren sowie von deren Additionssalzen mit einer pharmazeutisch annehmbaren Base.

15. Verfahren nach Anspruch 1 zur Herstellung von Verbindungen der Formel (I), in der $R_2$ eine durch eine Carbonsäuregruppe substituierte Niedrigalkenylgruppe darstellt, von deren Enantiomeren, Diastereoisomeren und Epimeren sowie von deren Additionssalzen mit einer pharmazeutisch annehmbaren Base.

16. Verfahren nach Anspruch 1 zur Herstellung von Verbindungen der Formel (I), in der $R_2$ eine Pyridylgruppe bedeutet, von deren Enantiomeren, Diastereoisomeren und Epimeren sowie von deren Additionssalzen mit einer pharmazeutisch annehmbaren Säure und, wenn $R_1$ ein Wasserstoffatom bedeutet, von deren Additionssalzen mit einer pharmazeutisch annehmbaren Base.

17. Verfahren nach Anspruch 1 zur Herstellung von Verbindungen der Formel (I), in der X ein Wasserstoffatom und Y eine Hydroxylgruppe bedeuten, von deren Enantiomeren, Diastereoisomeren und Epimeren sowie, wenn $R_1$ ein Wasserstoffatom darstellt, von deren Additionssalzen mit einer pharmazeutisch annehmbaren Base und, wenn $R_2$ eine stickstoffhaltige Gruppe umfaßt, von deren Additionssalzen mit einer pharmazeutisch annehmbaren Säure.

18. Verfahren nach Anspruch 1 zur Herstellung von Verbindungen der Formel (I), in der $R_2$ eine Niedrigalkylphenylgruppe bedeutet, von deren Enantiomeren, Diastereoisomeren und Epimeren sowie, wenn $R_1$ ein Wasserstoffatom darstellt, von deren Additionssalzen mit einer pharmazeutisch annehmbaren Base.

19. Verfahren nach einem der Ansprüche 1 oder 2 zur Herstellung von Verbindungen der Formel (I), in der $R_2$ eine Phenylgruppe bedeutet, die durch eine oder mehrere Gruppen, ausgewählt aus Niedrigalkylgruppen, Niedrigalkoxygruppen, Hydroxylgruppen und Trifluormethylgruppen, substituiert ist, von deren Isomeren, Diastereoisomeren und Epimeren sowie, wenn $R_1$ ein Wasserstoffatom bedeutet, von deren Additionssalzen mit einer pharmazeutisch annehmbaren Base.

20. Verfahren zur Herstellung von pharmazeutischen Zubereitungen, enthaltend als Wirkstoff mindestens eine Verbindung erhalten nach einem der Ansprüche 1 bis 19 in Kombination mit einem oder mehreren inerten, nichttoxischen, pharmazeutisch annehmbaren Bindemitteln oder Trägermaterialien.